(19)

Europäisches Patentamt
European Patent Office
Office européen des brevets

(11) **EP 3 403 572 A1**

(12) **EUROPEAN PATENT APPLICATION**

(43) Date of publication:
**21.11.2018 Bulletin 2018/47**

(21) Application number: **17171597.2**

(22) Date of filing: **17.05.2017**

(51) Int Cl.:
**A61B 5/024** (2006.01)      **A61B 5/00** (2006.01)
**A61B 5/16** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **My-Vitality SàRL**
**1297 Founex (CH)**

(72) Inventors:
• **DENNIS, John**
**1297 Founex (CH)**

• **NILCHIAN, Masih**
**1205 St. Sulpice (CH)**

(74) Representative: **KATZAROV S.A.**
**European Patent Attorneys**
**12, Avenue des Morgines**
**1213 Petit-Lancy (CH)**

Remarks:
Claims 18 to 22 are deemed to be abandoned due to non-payment of the claims fees (Rule 45(3) EPC).

(54) **PULSE WAVE DIAGNOSTIC DEVICE AND METHOD OF CALCULATING AND DETERMINING FATIGUE**

(57) The invention relates to a device, a system for the device and a set of methods used to extract pulse wave features and select an optimal combination of these features for calculating and determining the level of fatigue and fatigue related indicators of a subject. The device and its methods is meant to be used primarily for personal health care diagnosis and home use but can also be used by therapists, trainers and physicians to help them diagnose their patients and follow their patient's progress. The system is designed as a means of accurately obtaining, measuring, registering and interpreting the pulse to determine the level of energy or level of fatigue of a subject. By collecting pulse wave features, selecting those that are most significant and developing algorithms, the device and its method calculates the user's levels of fatigue and fatigue related indicators or personal energy.

FIG 10

EP 3 403 572 A1

**Description**

**FIELD OF THE INVENTION**

The invention relates to a device, a system for the device and a set of methods used to extract pulse wave features and select an optimal combination of these features for calculating and determining the level of fatigue and fatigue related indicators of a subject. The device and its methods is meant to be used primarily for personal health care diagnosis and home use but can also be used by therapists, trainers and physicians to help them diagnose their patients and follow their patient's progress. The system is designed as a means of accurately obtaining, measuring, registering and interpreting the pulse to determine the level of energy or level of fatigue of a subject. By collecting pulse wave features, selecting those that can be best used to differentiate between different sources of fatigue, and developing algorithms, the device and its method calculates the user's levels of fatigue and fatigue related indicators or personal energy.

**BACKGROUND OF THE INVENTION**

Physical and mental fatigue, and lack of sleep - also defined as fatigue related to sleep troubles - are three main sources of fatigue. They can often exist together even though they arise from different causes. Stress, anxiety, worry, depression or emotional grief can result in physical feelings of exhaustion even though the main source of fatigue is not from physical exertion. Similarly, extended periods of excess physical activity can result in feelings of stress and anxiety. Yet, it is hard sometimes to distinguish between these different sources of fatigue. This can lead to using wrong therapies even if the different sources of fatigue can manifest themselves in similar ways. If one is tired for emotional or mental reasons, going for a run could be a good remedy. Conversely, if one is physically fatigued from working out too much, getting extra rest for the body is no doubt a better therapy than working out.

Fatigue of a more chronic nature can be present because of many causes such as diseases, emotional trauma, living conditions/lifestyles and more specific situations and can be caused by so many various factors, diagnosis can be extremely difficult. In Western medicine, there is a tendency to seek out and try to cure specific physical ailments rather than try to cure more general health aspects of a patient. Thus, fatigue may be considered a secondary health problem that if even identified as a health issue might be considered a cause rather than an effect of an ailment.

Further compounding the problem is the fact that the patient's doctor or therapist has no objective means of identifying fatigue or determining the level of fatigue of a patient, or comparing it to other levels of fatigue of the patient over periods of time. Currently, a doctor or therapist will typically interrogate the patient to try to find the cause of fatigue, which could include the use of questionnaires. There are numerous problems with these interrogative techniques, including their reliability, effectiveness, subjectivity and ability to monitor progress. Questionnaires are considerably subjective and prone to considerable error. There is no way to tell how truthful the responses are, how much thought a respondent has put into it, nor if the respondent has interpreted the questions in the right manner. Answers are influenced by moods and emotions at that time of the self-assessment and vary in the weights placed on their meaning. Fatigue is often a subjective feeling reported by the individual rather than an objective one. Fatigue and subjective feelings of fatigue are often confused.

In Traditional Chinese Medicine (TCM), and in many other areas of the world including Japan, India, Korea and the Middle East, therapists/physicians also use similar enquiry methods as in the West to seek medical solutions. Unlike in Western medicine, they may also rely on taking the pulse as a means of helping determine the patient's overall health (beyond examining pulse rates and blood pressure). Pulse taking as practiced in TCM is primarily not to determine pulse rates and pulse rhythm as in Western medicine, but rather performed for the purpose of examining the conditions of the whole body and to detect disease and organ malfunctioning. TCM derived pulse type classifications are complex and difficult to understand. Pulse classifications are difficult to articulate from finger sensations and include such classifications as "floating, replete, sunken, slippery, hollow, rough," or the like. These descriptive classifications are difficult to use for comparisons over time or among different persons.

A challenging aspect to developing a system to measure fatigue is the lack of reference(s) or a gold standard or biomarkers from which to cross reference estimates of fatigue levels. There is no way to verify in a quantifiable and objective manner calculated fatigue levels or estimates or predictions of fatigue. Without such standards, it is difficult to build mathematical or analytical models without the corresponding correlations. It is difficult to verify such a system, if the results cannot be compared with other numerical values for fatigue.

Within the more general area of fatigue, there are more specific sources of fatigue where there is also need for measurement and monitoring. Sports training is an area where it is difficult to measure and monitor an athlete's level of fatigue. An athlete seeks to increase his/her training load to improve performance. However, in the process of pushing towards peak performance, the athlete risks a state referred to as "over-reach", a state where the athlete's overload can be detrimental to the training program. Short term overload, as described by sports specialists, can be managed with a few days of extra rest. However, overreaching at higher levels can develop into over-training, where it is considerably more difficult to recover and may result in a loss of weeks or even months of training to recover. Accordingly, it is important that an athlete's training program can be monitored and its effects measured to help ensure that fatigue of a more destructive nature does not occur.

Some potential markers are available to athletes, coaches and scientists to help monitor training loads. However, few if any of these markers show strong evidence supporting their use, especially among athletes who are not professionally coached, and there is still no single and reliable marker of this physical type of fatigue. Performance testing where variances on performance are measured is a popular way of trying to detect differences in the athlete's physical and mental state. The problem is that performance testing is difficult to carry across different types of training such as between endurance-focused training and training focused on building muscle. This problem is also prevalent with tracking devices that can measure distances run or walked based on body motion but are not able to translate this into more stationary training such as muscle building. Another problem is trying to consider the effects of timing and training intensity and its influence on performance. Training has an accumulative effect over extended periods and varies as a function of rest periods and training duration. Further, while external loads can be measured to some extend through measuring body activity it does not consider various internal body factors such as physiological and psychological influences.

Measurements of Heart Rate Variability (HRV) is proposed as an indication of both positive and negative adaptations to training. A relatively large amount of research has been conducted on HRV. However, the varying methodologies employed as well as the high day-to-day variability in environmental and homeostatic factors have resulted in inconsistent findings. HRV is confined primarily to measuring the heart's electrical system. It does not consider various physiological factors such as breathing, blood flow, and other cardiovascular properties such as vasoconstriction and vasodilation.

Lack of sleep is also an important source of general fatigue. Yet, the effects of sleep deprivation on overall feelings of fatigue and how they differ from physical fatigue are not well known. Sleep clinics offer sleep scoring as a way of measuring the amount and quality of sleep. This usually involves the subject wearing polysomnogram (PSG) equipment primarily on and around the head and usually in a clinic. This process is difficult to administer and uncomfortable to wear overnight. The data usually must be analyzed afterwards by a technician, further making this process expensive. Wearable devices are also available to measure sleep quality. While these measurements are good, their accuracy can be improved.

While improvements to measuring sleep-sourced fatigue are needed, the main problem is that there is no objective way of knowing in a measurable way the effects of lack of sleep on overall states of fatigue. Without knowing this interrelationship, it is difficult to measure fatigue levels. Again, there are no standards that can be used to verify whether a system that measures fatigue is accurate.

Mental fatigue is a temporary inability to maintain optimal cognitive performance. Mental fatigue can be caused by continual mental effort and attention on a single or set of tasks, as well as high levels of stress or emotion. A mentally demanding exercise that goes into overload (i.e. brain over-activity) can result in the brain cells becoming exhausted. It is difficult to find reliable and easy-to-use objective measurements of cognitive fatigue. Besides questionnaires and cognitive tests, efforts have been used to measure brain activity through electroencephalography (EEG) equipment. EEG equipment is also difficult to use as it involves attaching electrodes to the scalp and sending the generated brain signals to a computer. It requires considerable knowledge to understand the data generated, making the system relatively difficult for home use.

Since mental tiredness is a source of general fatigue, it needs to be considered when measuring overall fatigue. Currently, there are no mentally sourced fatigue standards that can be used to verify whether a system that measures fatigue is accurate. It is generally known that when one is mentally tired that one's reaction time to external stimuli is slowed. It is also known that one's mental stamina to perform tasks is shortened when tired. Accordingly, there is also the problem of finding gold standards that can be used to verify fatigue assessments.

Fatigue is one of the most commonly encountered complaints in describing a medical condition or illness. It is associated with many medical conditions such as hypothyroidism, celiac disease, anemia, influenza, heart failure, and sleep apnea. It is the largest symptom in chronic fatigue syndrome (CFS) and chemotherapy. Fatigue may also be caused by the remedies used to help cure an illness. Many pharmacological products warn that their products can result in tiredness and fatigue. Currently, it is difficult to measure and monitor the resulting levels of fatigue in an objective manner. An objective quantification of levels of fatigue could enhance the therapy practices used in the illness remedy. Certainly, not knowing levels of tiredness complicates the correct administration of therapy and dosage. Overall, the patient's health outcomes would be improved if levels of fatigue were monitored on a regular basis thereby improving personal treatment and ensuring that personal treatment is better adapted.

It is also difficult to make a system with a device and a set of procedures used with the device for diagnosing the level of fatigue of an individual without being able to verify its effectiveness. Without standards to reference and correlate against, it is difficult to know how good such a system is at indicating fatigue levels associated or not associated with another ailment.

Attempts have been made to develop more objective and standardized pulse diagnosis. With the advent of sensor technology along with supporting sensor technologies, progress has been made in the quantification and less subjective interpretation of pulse diagnosis. There have been attempts to replace finger sensation with pulse reading devices. This includes, more recently, technology that has shifted single element to multi element sensors, which provide more accurate information. In addition, numerous attempts have been made to simplify the complexity of pulse interpretation in various publications.

Various examples of this are described for example in EP 0835 633 A2 (MARUYA TOKINORI) relating to a device which is described as sensor belts that are wrapped around the patient's wrists. The apparatus converts the signals picked up by the sensors into pulse waves. The apparatus stores the acquired data in memory and provides, in turn, the data on a monitor in various display and print formats.

In US 5,381,797 (PAK SONG C et al.) a pulse wave signal processing unit is described using a pen like device which is pressed down on the three pulse taking sites on the radial artery. The device includes an amplifier and a means of correcting its amplitude-frequency response to obtain a shape from the pulse wave signal.

In US 8,317,716 (KIM JONG YEOL et al.) a pulse taking device is described that picks up pulse signals using several sensors. In addition, the invention embodies an automatic diagnostic system capable of determining pulse-taking locations. Improvements are also made on technically improving the efficiency of processing the pulse signals. These devices also attempt to quantify the attained pulse data by way of graphs and formulas.

However, these devices do not address the main purpose of the present invention, namely measuring or quantifying an individual's level of fatigue. Rather, these devices attempt to produce a large array of data to help the doctor or therapist make medical diagnosis of various health ailments. These inventions provide a large array of data used to categorize pulses into as many as 28 different pulse types as practiced in TCM.

In JPH08299292A (SEIKO EPSON CORP.) no specifics are provided in extracting or selecting pulse wave features or the use of specific pulse wave characteristics and its relation to measuring various aspects of personal energy levels.

Similarly, in US 2004/0181159 (KUO TERRY B J et al.) methods are provided for measuring yin and yang parameters but no pulse wave details are provided for the diagnosis or determination of personal energy levels.

Thus, the main problem of the invention is that it is difficult to measure levels of fatigue because of a lack of standards needed to make and verify these measurements. There are no single sets of biomarkers or other standards since there are different causes of fatigue and because fatigue or feelings of fatigue manifests itself in different ways.

## BRIEF DESCRIPTION OF THE INVENTION

One of the objects of the present invention is to an exemplary embodiment, a method of calculating and determining the level of fatigue or fatigue-related indicators in a subject having previously undergone a set of pulse wave recordations, the level of fatigue selected from among physical fatigue, mental fatigue, fatigue related to lack of oxygen, fatigue related to sleep troubles, fatigue related to stress, or a combination of two or more of the above, may be provided.

Such a method may include the steps of: extracting and selecting from a single pulse wave and from its first and second derivation a first set of features selected among time, amplitude, area, ratios, heart rate and breathing rate; performing a statistical analysis on said first set of features obtained from at least two pulse waves to arrive at a second set of features selected among mean, variation around mean, and randomness; and combining said first and second set of features and applying means configured in a software to analyse, determine and display results of fatigue or fatigue related indicators of said subject.

According to another exemplary embodiment, a pulse wave diagnostic device for determining and quantifying the level of fatigue or fatigue-related indicators in a subject, the level of fatigue selected from among physical fatigue, mental fatigue, fatigue related to lack of oxygen, fatigue related to sleep troubles, and fatigue related to stress, or a combination of two or more of the above. The pulse wave diagnostic device may be applied on a pulse-taking location on the body of said subject. The pulse wave diagnostic device may be configured to perform the functions of extracting and selecting from a single pulse wave and from its first and second derivation a first set of features selected among time, amplitude, area, ratios, heart rate and breathing rate; performing a statistical analysis on said first set of features obtained from at least two pulse waves to arrive at a second set of features selected among mean, variation around mean and randomness; and combining said first and second set of features and transmitting these signals to a software configured to analyse and display results of the level of fatigue or fatigue related indicators of said subject.

Other objects and advantages of the invention will become apparent to those skilled in the art from a review of the ensuing detailed description, which proceeds with reference to the following illustrative drawings, and the attendant claims.

**BRIEF DESCRIPTION OF THE FIGURES**

Advantages of embodiments of the present invention will be apparent from the following detailed description of the exemplary embodiments thereof, which description should be considered in conjunction with the accompanying figures in which like numerals indicate like elements, in which:

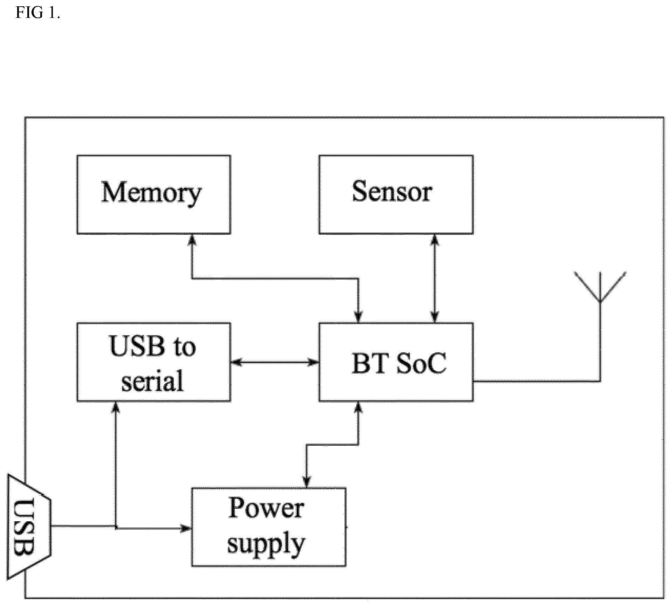

**FIG 1** is an exemplary embodiment of a circuit diagram showing an example of some of the main components in a circuit configuration of a pulse wave extraction and recording device. Specifically, FIG. 1 depicts, from top to bottom and left to right: a memory, a sensor, a USB to serial adapter, a Bluetooth antenna, a USB power supply, and a battery.

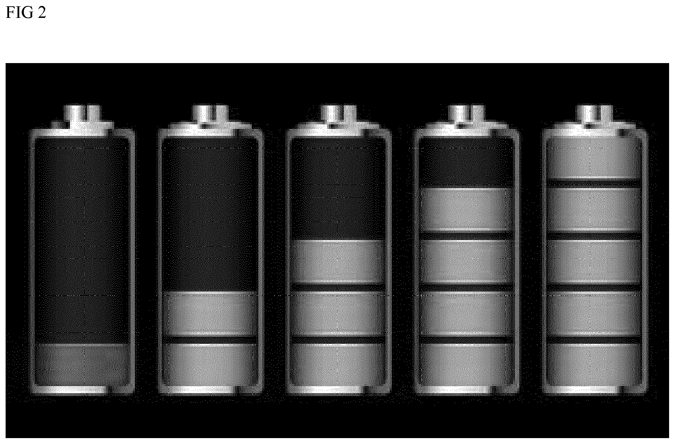

**FIG 2** is an exemplary embodiment of a visual image of a battery, which may be provided as a way of depicting in an easily understandable way the level of fatigue.

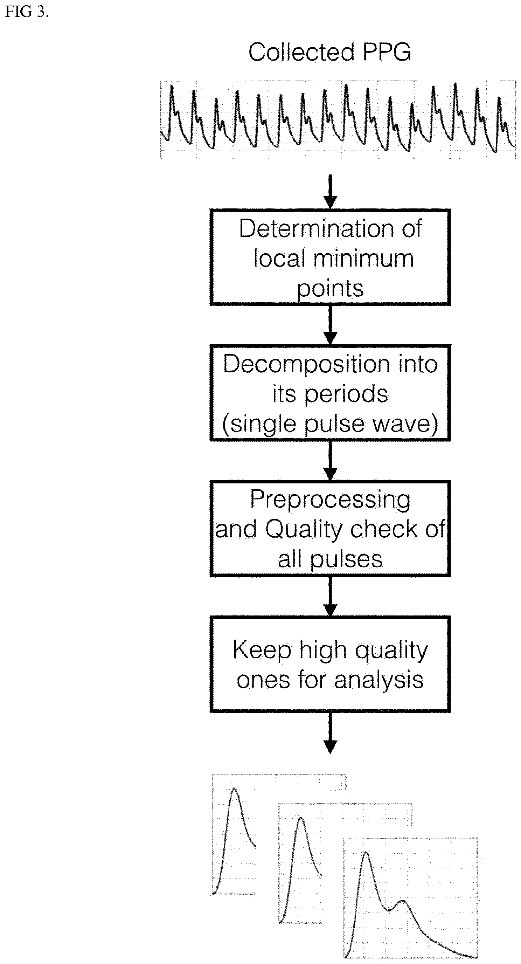

**FIG 3** is an exemplary embodiment of a diagram in a set of modules which may show a method for collecting pulse waves for a period of time and identifying a set of individual pulse waves of quality.

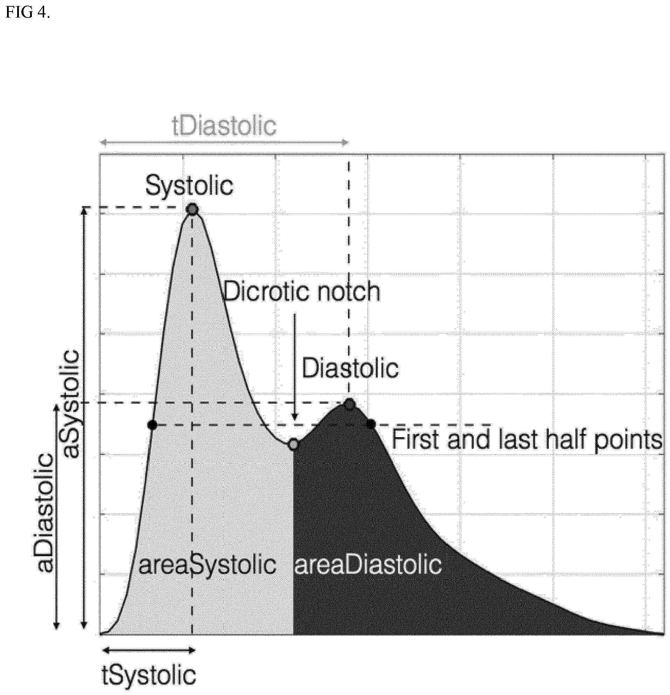

**FIG 4** is an exemplary embodiment of a diagram of a single pulse wave which may depict a systolic peak, a diastolic peak, a dicrotic notch, the first and the last points corresponding to the half-height of the systolic peak with their times, and amplitudes of the single pulse wave.

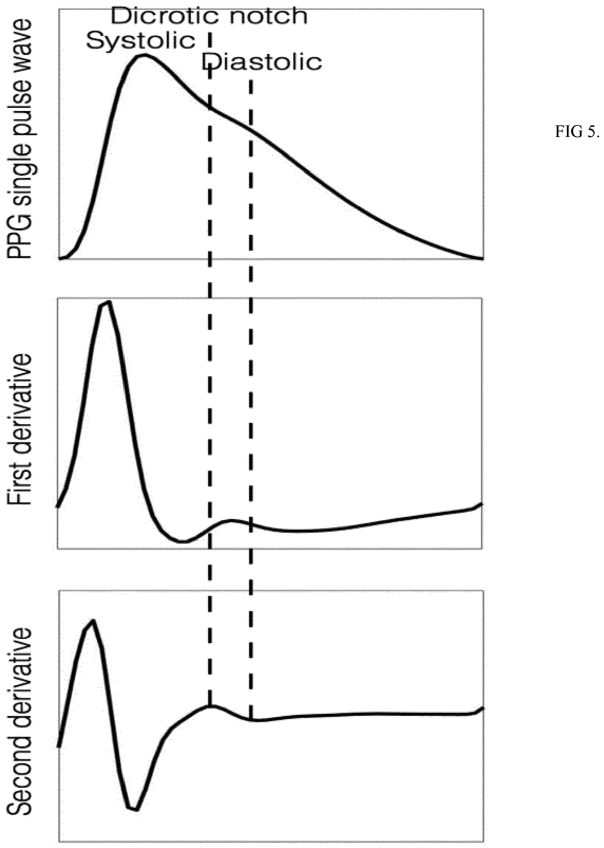

**FIG 5** is an exemplary embodiment of a diagram of a pulse wave whose diastolic peak is challenging to identify. It also depicts its first and second derivative curves. The diastolic peak and the dicrotic notch is identified using the second derivative of the pulse wave.

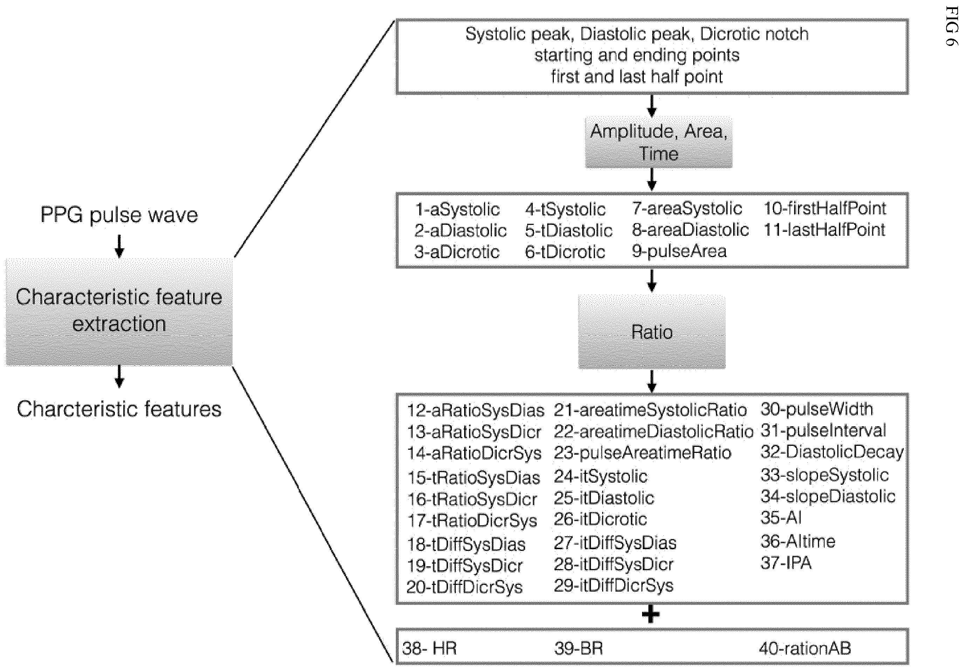

**FIG 6** is an exemplary embodiment of a diagram in a set of modules which may show the method by which the first set of features of pulse wave (characteristic features) are obtained from the pulse wave timeline and its seven points: systolic peak, diastolic peak, dicrotic notch, starting and ending point, and the first and the second points corresponding to the half-height of the systolic peak. Original features may be obtained from the pulse wave by applying the calculations of time, amplitude, area, and ratios.

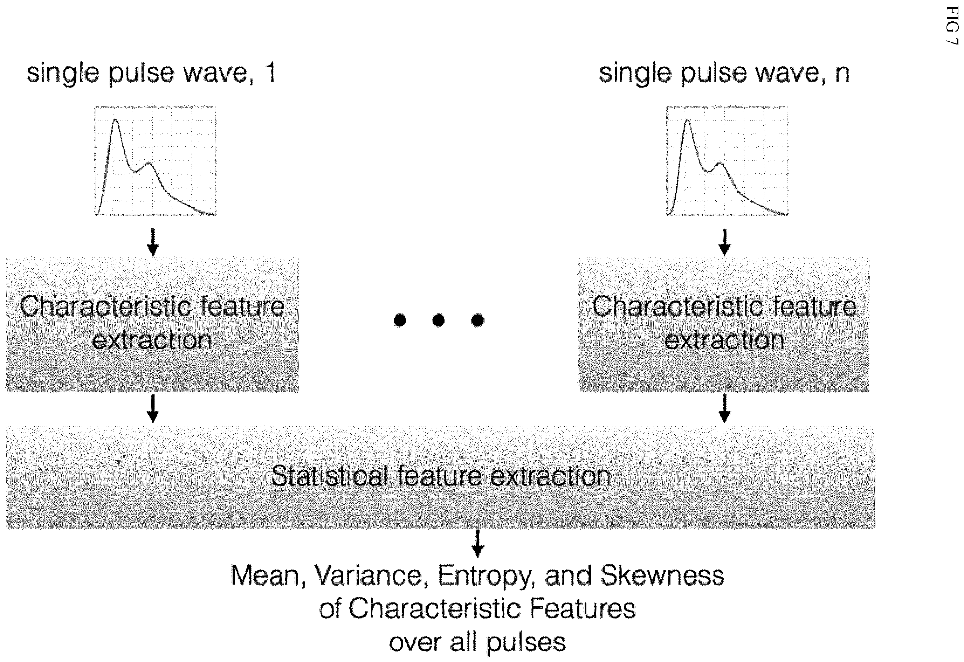

**FIG 7** is an exemplary embodiment of a diagram depicting a final step in the illustrated method of FIG. 6. As a final step in this illustrated method, the second set of features may be obtained by calculating, for each feature in the first set of features, its respective mean, variance, skewness and entropy.

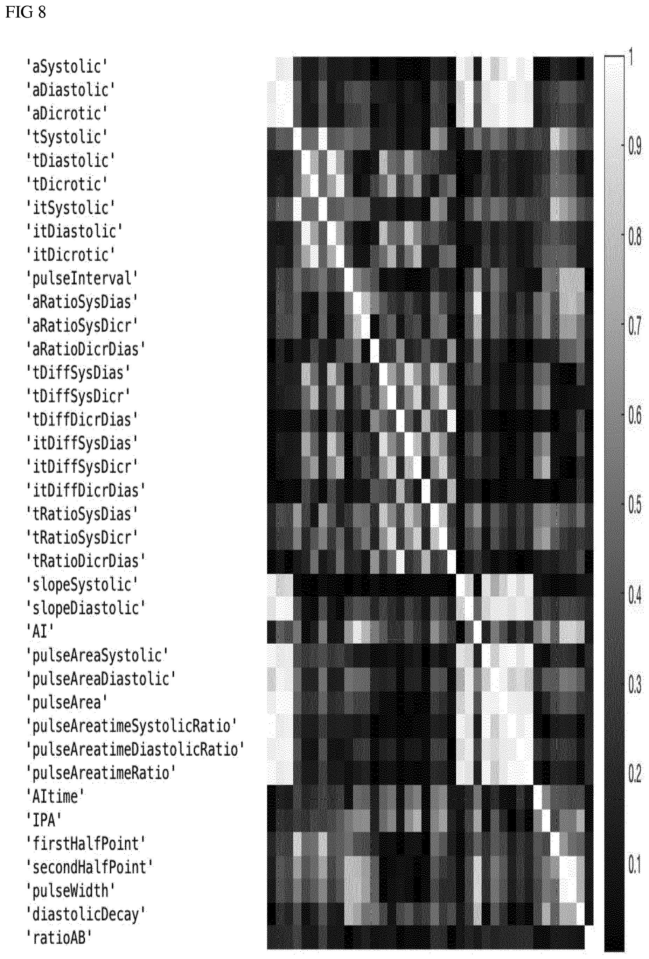

**FIG 8** is an exemplary illustration of the correlation between two features. The darker images on the grayscale presents those combinations of features that are independent or complementary from each other. Conversely, lighter images depict higher levels of inter-relationship.

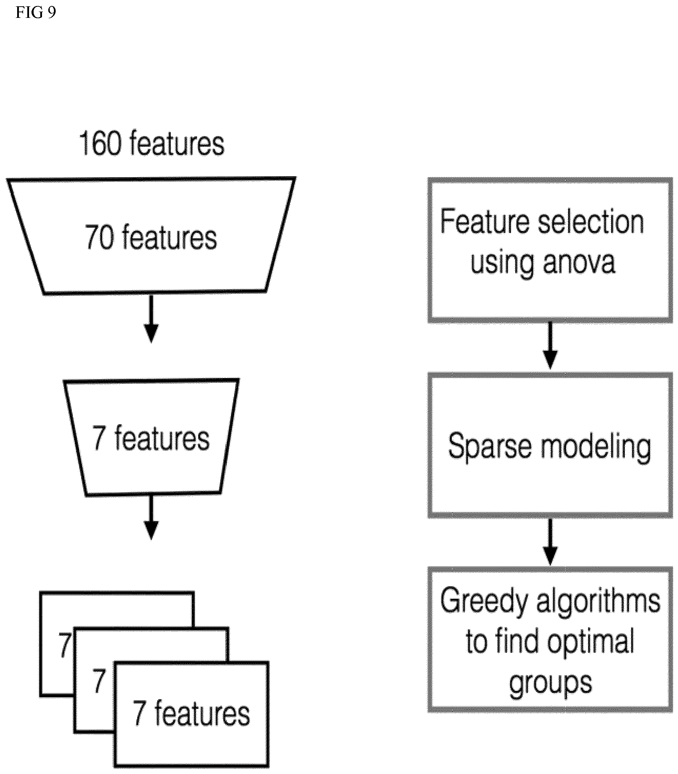

**FIG 9** is an exemplary embodiment of a diagram showing a much-simplified illustration of the methodology used to obtain an optimal set or group of features as an indication of levels of fatigue. The anova math method including the F-test technique may be used to identify the pulse wave features most useful to differentiate between levels of fatigue. The method purposes to narrow down the number of features to around 70. From these 70 features, various sparse math techniques are used to identify sub-sets or groups of features best permit differentiation. Upon the identification of around 20 sets or combinations of features that show correlation with various aspects of the levels of fatigue, the features in each group are replaced one by one with the other features to continue to get the best sub-sets of features. By repeating these steps a few times such as five times, a best group or optimal sub-sets or combination of features are identified.

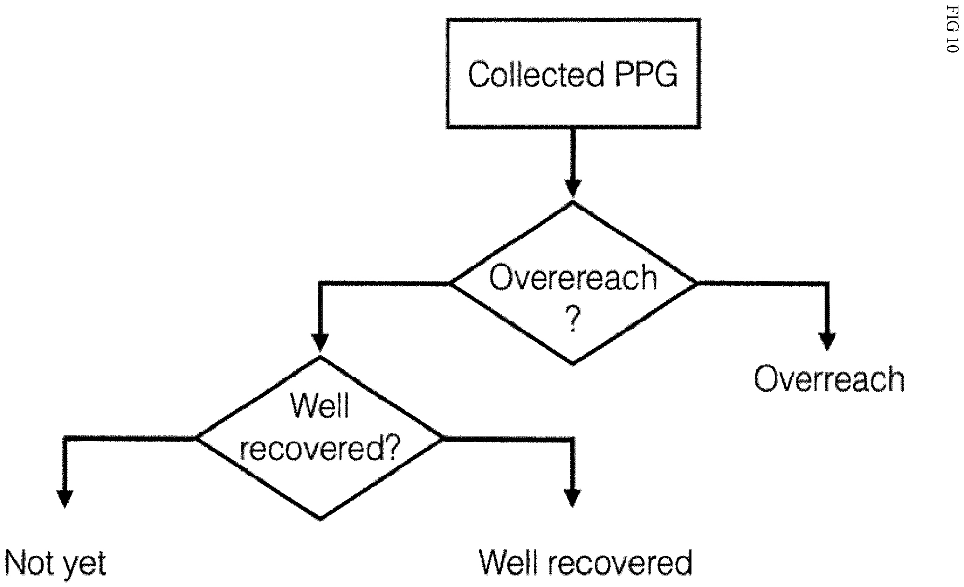

**FIG 10** is an exemplary embodiment of a diagram showing the decision tree to first distinguish overreach from non-overreach, and then, for conditions of non-overreach, distinguish between patients who are well-recovered versus those are not well-recovered.

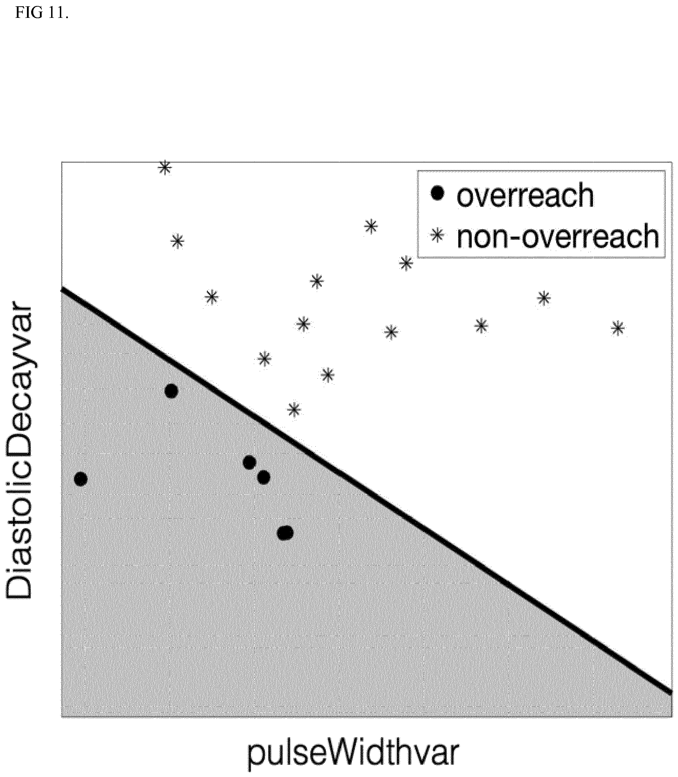

**FIG 11** is an exemplary embodiment of a scatter plot that illustrates the possibility of classification of the subjects into overreach and non-overreach using variance of the diastolic decay and the variance of the pulse width.

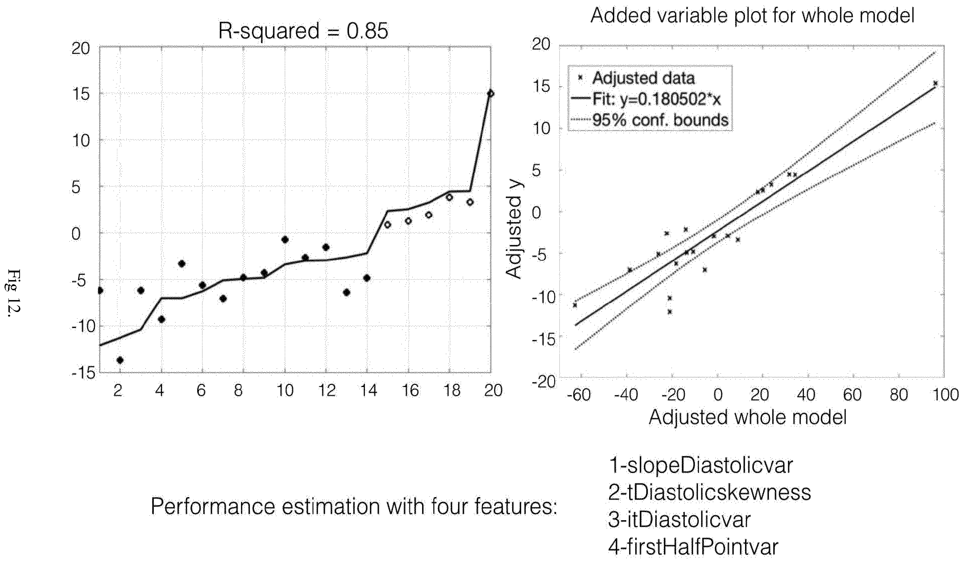

**FIG 12** is an exemplary embodiment of a diagram illustrating prediction of a change in 3km run test performance using a regression of the variance of the diastolic decay, the skewness of the diastolic time, the variance of the inverse of diastolic time, and the variance of the first point corresponding to the half-height of the systolic peak.

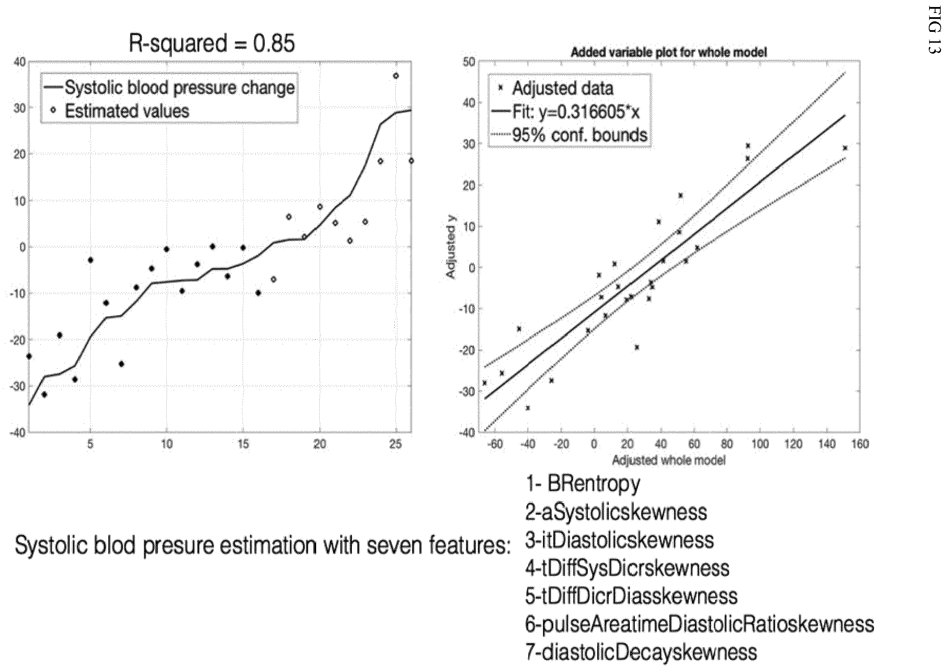

**FIG 13** is an exemplary embodiment of a diagram illustrating prediction of a change in systolic blood pressure using a regression of the entropy of the breathing rate, the skewness of the systolic amplitude, the skewness of the inverse of diastolic time, the skewness of the time difference between the systolic peak and the dicrotic notch, the skewness of time difference between the dicrotic notch and the diastolic peak, the skewness of the ratio of the area under curve between the starting point and dicrotic notch by the dicrotic notch time, and the skewness of diastolic decay.

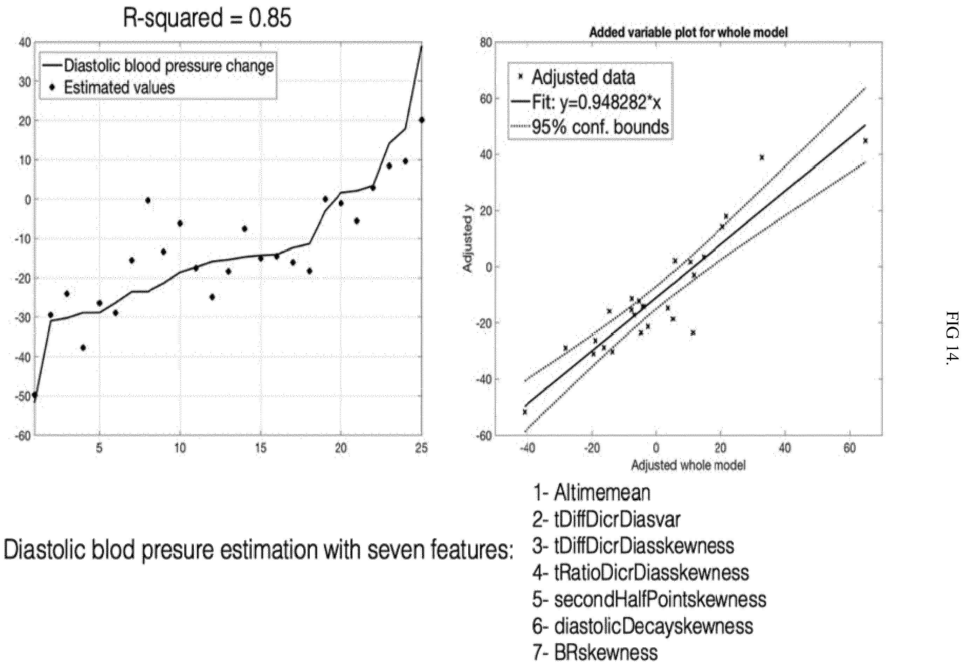

**FIG 14** is an exemplary embodiment of a diagram illustrating prediction of a change in diastolic blood pressure using a regression of the mean of augmentation index, the variance of time difference between the dicrotic notch and the diastolic peak, the skewness of time difference of between the dicrotic notch and the diastolic peak, the skewness of the ratio of the dicrotic notch time to the diastolic time, the skewness of diastolic decay, and the skewness of the breathing rate.

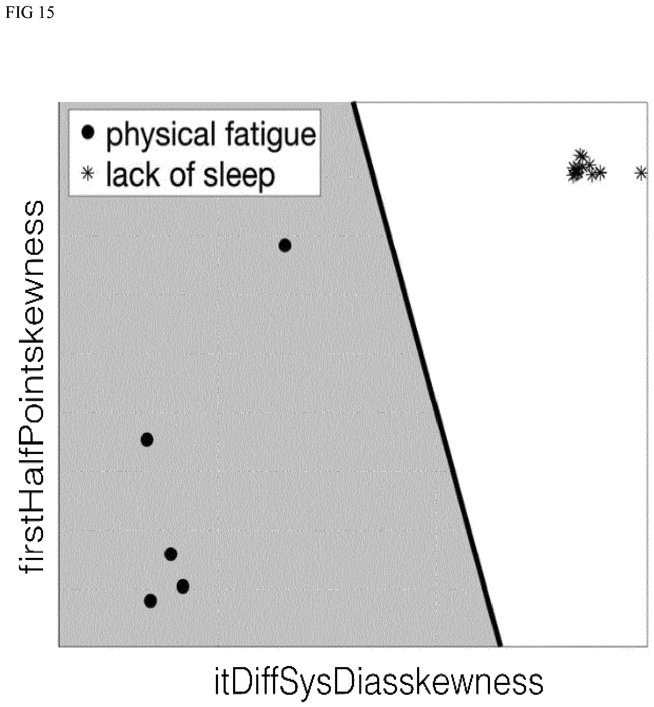

**FIG 15** is an exemplary embodiment of a scatter plot that illustrates the possibility of classification of the subjects into physical fatigue and fatigue caused by lack of sleep using the skewness of the first point corresponding to the half-height of the systolic peak and the skewness of the inverse of time difference between the systolic and diastolic peaks.

## DETAILED DESCRIPTION OF THE INVENTION

Although methods and materials similar or equivalent to those described herein can be used in the practice or testing of the present invention, suitable methods and materials are described below. All publications, patent applications, patents, and other references mentioned herein are incorporated by reference in their entirety. The publications and applications discussed herein are provided solely for their disclosure prior to the filing date of the present application. Nothing herein is to be construed as an admission that the present invention is not entitled to antedate such publication by virtue of prior invention. In addition, the materials, methods, and examples are illustrative only and are not intended to be limiting. It should be understood that the described embodiments are not necessarily to be construed as preferred or advantageous over other embodiments. Moreover, the terms "embodiments of the invention", "embodiments" or "invention" do not require that all embodiments of the invention include the discussed feature, advantage or mode of operation.

In the case of conflict, the present specification, including definitions, will control.

Unless defined otherwise, all technical and scientific terms used herein have the same meaning as is commonly understood by one of skill in art to which the subject matter herein belongs. As used herein, the following definitions are supplied in order to facilitate the understanding of the present invention.

The term "comprise" is generally used in the sense of include, that is to say permitting the presence of one or more features or components.

Some embodiments may be described in terms of sequences of actions to be performed by, for example, elements of a computing device. It will be recognized that various actions described herein can be performed by specific circuits (e.g., application specific integrated circuits (ASICs)), by program instructions being executed by one or more processors, or by a combination of both. Additionally, these sequence of actions described herein can be considered to be embodied entirely within any form of computer readable storage medium having stored therein a corresponding set of computer instructions that upon execution would cause an associated processor to perform the functionality described herein. Thus, the various aspects of the invention may be embodied in a number of different forms, all of which have been contemplated to be within the scope of the claimed subject matter. In addition, for each of the embodiments described herein, the corresponding form of any such embodiments may be described herein as, for example, "logic configured to" perform the described action.

As used in the specification and claims, the singular forms "a", "an" and "the" include plural references unless the context clearly dictates otherwise.

As used herein the terms "subject" or "patient" or "individual" are well-recognized in the art, and, are used interchangeably herein to refer to a mammal, including dog, cat, rat, mouse, monkey, cow, horse, goat, sheep, pig, camel, and, most preferably, a human. In some embodiments, the subj ect is a subj ect in need of treatment or a subj ect with a disease or disorder. However, in other embodiments, the subject can be a normal subject. The term does not denote a particular age or sex. Thus, adult and newborn subjects, whether male or female, are intended to be covered.

A "pulse wave" (PW) is the progressive increase of pressure radiating through the arteries that occurs with each contraction of the left ventricle of the heart. In other words, a pulse wave (PW) is a measure of the change in the volume of arterial blood with each pulse beat. Specifically, the arterial pulse waveform is a contour wave generated by the heart when it contracts, and it travels along the arterial walls of the arterial tree. Generally, there are 2 main components of this wave: a forward moving wave and a reflected wave. The forward wave is generated when the heart (ventricles) contracts during systole. This wave travels down the large aorta from the heart and gets reflected at the bifurcation or the "cross-road" of the aorta into 2 iliac vessels. In a normal healthy person, the reflected wave usually returns in the diastolic phase, after the closure of the aorta valves. The returned wave which gives a notch pushes the blood through the coronaries. As shown in Figure 4, seven main timeline points can be used to obtain pulse wave features: (1) starting point, (2) first point corresponding to the half-height of the systolic peak (3) Systolic peak (4) Dicrotic notch (5) Diastolic peak and (6) last point corresponding to the half-height of the systolic peak and (7) ending point.

"Fatigue" may also be referred to in such terms as exhaustion, weakness, lethargy, tiredness, describe a general physical and/or mental state of being or feeling weak, lacking energy, lacking vitality, zeal or zest, lacking strength, apathy, feeling "often tired", etc. Fatigue is one of the most commonly encountered complaints in medical practice. In Western medicine, it is characterized by feelings of low levels of energy, a lessened capacity or motivation to work or be active, and often accompanied by sleepiness and weakness. In Chinese Traditional Medicine (TCM) and other oriental medicine, they refer to this condition as lacking Qi or lacking energy. Qi is considered generally your life force or vital energy, which circulates in and around all of us. This Qi can stagnate or be blocked and a significant part of TCM involves "unblocking" or releasing this Qi.

Physical and mental fatigue and lack of sleep also referred herein as fatigue related to sleep troubles are three main sources of fatigue. They can often exist together even though they arise from different causes. Stress, anxiety, worry, depression or emotional grief can result in physical feelings of exhaustion even though the main source of fatigue is not from physical exertion. Similarly, extended periods of access physical activity can result in feelings of stress and anxiety. The result is that an individual will have a general feeling of tiredness of a more chronic nature than a short term feeling of exhaustion, such as might be caused by, for example, a lack of sleep or a lot of physical exercise. With a general feeling that one has a lack of energy reserves or that the "battery is low", such tiredness can manifest itself in such emotional states as lethargy, lack of ambition or even have a direct effect physically such as a weakness of the immune system, making one more prone to colds/flues or other ailments.

Within the more general area of fatigue, there are more specific sources, indicators or factors of fatigue where there is also need for measurement and monitoring. Those "fatigue related indicators" or factors are selected among physical fatigue, mental fatigue, fatigue related to lack of oxygen, fatigue related to sleep troubles, fatigue related to stress or a combination thereof. In particular, physical fatigue may include overload, performance, discrimination or differentiation between overreach and non-overreach in sports activity and differentiation between a well-recovered state and a non-well-recovered state in sports activity. On the other hand, fatigue related to sleep troubles may include somnolence or drowsiness, sleep deprivation, sleep efficiency and a lack of deep sleep and/or REM (Rapid Eye Movement).

Sports training is an area where it is difficult to measure and monitor an athlete's level of fatigue i.e. physical fatigue. An athlete seeks to increase his/her training load in order to improve "performance". Performance is defined as the fact of carrying out of specific physical routines or procedures by one who is trained or skilled in physical activity. Performance is influenced by a combination of physiological, psychological, stress and socio-cultural factors.

However, in the process of pushing towards peak performance, the athlete risks entering into a state referred to as "overreach", a state where the athlete's overload can be detrimental to the training program and performance.

"Overload" is meant to describe a state where one has exerted oneself or burdened oneself physically more than usual or normal thereby possibly causing physical tiredness.

In sport, "overload" means that to improve, athletes must continually work harder as they their bodies adjust to existing workouts. Short term overload, as described by sports specialists, can be managed with a few days of extra rest. However, overreaching at higher levels can develop into over-training, where it is considerably more difficult to recover and may result in a loss of weeks or even months of training to recover. Accordingly, it is important that an athlete's training program can be monitored and its effects measured to help ensure that fatigue of a more destructive nature does not occur. It is important that someone in training knows when he is in "non-overreach" and when he is or is at risk of being or going into overreach.

The Recovery Principle dictates that athletes need adequate time to recuperate from training and competition. Many believe that an athlete's ability to recover from workouts is just as important as the workout itself. It is during rest periods that athletes' bodies adapt to the stress placed upon them during intense workout sessions and competitions. Rest also provides time for a mental preparation and reflection. The Recovery Principle applies both to immediate rest needed between bouts of exercise, as well as to longer time intervals of several hours to about two days. "Well-recovered" means to become completely well again after fatigue arising from sports activity, the latter being the opposite of "non-recovered".

"Sleep disorders" can be a source of fatigue. Sleep troubles or sleep disturbance or disorders may represent any disorders excluding environmental factors (such as noise, movement, travel through time zones, or change in altitude) that affect, disrupt, or involve sleep. The most common sleep disorder is probably snoring, although it is usually not medically significant. Insomnia, sleep apnea, restless leg syndrome, and sleepwalking are also sleep disorders. Generally, sleep disturbances encompass disorders of initiating and maintaining sleep (DIMS, insomnias), disorders of excessive somnolence (DOES), disorders of sleep-wake schedule, and dysfunctions associated with sleep, sleep stages, or partial arousals (parasomnias). A lack of deep sleep and/or REM is also a source of fatigue, as is wakefulness caused by stress.

"Somnolence" or drowsiness or hypersomnia is a syndrome when people feel very sleepy during the day or want to sleep for longer than normal at night, and may often be ready to fall asleep or dull with sleepiness. Somnolence may also be called excessive daytime sleepiness, or prolonged drowsiness. Somnolence or drowsiness is also a state preceding falling asleep, which can be dangerous if one is driving or in situations where alertness is needed for reasons such as combat, surveillance or for manual or mental interventions (for example surgery). It is considered the phase between wakefulness and sleep.

"Sleep deprivation" or lack of sleep is also an important source of general fatigue. Sleep deprivation is a condition that occurs if a subject doesn't get enough sleep. Sleep deficiency is a broader concept.

Sleep deficiency can occur when one has one or more of the following. Sleep deficiency can occur when one doesn't get enough sleep (sleep deprivation); one sleeps at the wrong time of day (i.e. out of sync with body's natural clock); one doesn't sleep well or get all of the different types of sleep that the body needs; or one has a sleep disorder that prevents from getting enough sleep or causes poor quality sleep. Sleeping is a basic human need, like eating, drinking, and breathing. Like these other needs, sleeping is a vital part of the foundation for good health and well-being throughout the subject's lifetime. Sleep deficiency or lack of sleep efficiency can lead to physical and mental health problems, injuries, loss of productivity, and even a greater risk of death. Yet, the effects of sleep deprivation on overall feelings of fatigue and how they differ from physical fatigue are not well known.

"Sleep efficiency" is the ratio of the total time spent asleep (total sleep time) in a night compared to the total amount of time spent in bed. For example, if a man spends 8 hours in bed on a given night, but only actually sleeps for four of those hours, his sleep efficiency for that evening would be 50% (four divided by eight multiplied by 100 percent). As another example, a woman who sleeps six out of the 8 hours spent in bed would have a sleep efficiency of 75% (six divided by eight multiplied by 100 percent). If an individual spends the majority of the time that they are in bed actually asleep, then they are considered to be sleep efficient (or to have a high sleep efficiency). However, if an individual spends a lot of the total time that they are in bed awake, then that is not considered to be sleep efficient (or the person has a low sleep efficiency). An efficient sleep leads to a deeper sleep of higher quality with fewer interruptions. It may result in feelings of energy and being well-rested upon awakening, while an inefficient sleep may lead to feelings of tiredness and restlessness.

Fatigue can also be a result of a lack of deep sleep and/or REM. During deep sleep stage, it is harder to rouse. This is the stage where the body repairs and regrows tissue, builds bone and muscle and strengths the immune system. One normally has intense dreams during the rapid eye movement or REM stage since the brain is more active. During REM while the brain is more active most muscles move very little.

"Heart Rate Variability" (HRV) is the physiological phenomenon of variation in the time interval between heartbeats. It is measured by the variation in the beat-to-beat interval. Other terms used include: "cycle length variability", "RR variability" (where R is a point corresponding to the peak of the QRS complex of the ECG wave; and RR is the interval between successive Rs), and "heart period variability". Measurements of Heart Rate Variability (HRV) is proposed as an indication of both positive and negative adaptations to training and as a potential partial indicator of fatigue and stress. To measure fatigue and fatigue related indications it is important to not only estimate HRV but to be able to predict HRV based on a combination of features extracted and selected from PPG.

Blood pressure (BP) is the pressure of circulating blood on the walls of blood vessels. When used without further specification, blood pressure usually refers to the pressure in large arteries of the systemic circulation. Normal fluctuation in blood pressure or "blood pressure change" is adaptive and necessary. Studies have shown, for example, that a lack of sleep can limit the body's ability to regulate stress hormones, leading to higher blood pressure. The absolute values of systolic pressure and diastolic pressure compared to a baseline can be an indicator of fatigue. It is therefore helpful to get indications of these pressures through the combination of features from PPG.

It is also known from other studies that the automatic nervous system regulates the size of arteries. The baroreflex or baroreceptor reflex is one of the body's homeostatic mechanisms that helps to maintain blood pressure at nearly constant levels. Baroreflex induced changes in blood pressure are mediated by both branches of the autonomic nervous system - that is the parasympathetic and sympathetic nerves. Baroreceptors are active even at normal blood pressures so that their activity informs the brain about both increases and decreases in blood pressure. Accordingly, extended levels of stress can lead to imbalances in levels of blood pressure, an indicator of fatigue and stress.

"Mental fatigue" or cognitive fatigue is a temporary inability to maintain optimal cognitive performance. It is defined by a condition of low alertness or cognitive impairment, usually associated with prolonged mental activities or stress. In particular, mental fatigue can be caused by continual mental effort and attention on a single or set of tasks, as well as high levels of stress or emotion. A mental demanding exercise that goes into overload (i.e. brain over-activity) can result in the brain cells becoming exhausted. Since mental tiredness is a source of general fatigue, it needs to be considered when measuring overall fatigue. In addition, when mentally tired, one's reaction to external stimuli is slowed and one's ability to perform protracted mental tasks is shortened.

A "lack of oxygen" can be a source of fatigue. For example, an important factor in Acute Mountain Sickness (AMS) is fatigue caused by lower levels of oxygen and decreased air pressure. Roughly half of all people who stay suddenly at altitudes of over 3,000 meters for a number of hours or overnight get various forms of AMS. The speed which one rises in altitude and the length of time one is at higher altitudes and respiration rates are factors influencing AMS. It is currently difficult to estimate or predict AMS. Yet many mountaineers risk AMS, which can result in migraine headaches, nausea and fatigue. Since a lack of oxygen or changes in air conditions (i.e. change in pressure) is a source of general fatigue it needs to be considered when measuring overall fatigue.

"Stress" is a physical, mental, or emotional factor that causes bodily or mental tension. Stresses can be external (from the environment, psychological, or social situations) or internal (illness, or from a medical procedure). Stress can initiate the "fight or flight" response, a complex reaction of neurologic and endocrinologic systems. Catecholamine hormones, such as adrenaline or noradrenaline, facilitate immediate physical reactions associated with a preparation for violent muscular action. These include the following: acceleration of heart and lung action; paling or flushing, or alternating between both; inhibition of stomach and upper-intestinal action to the point where digestion slows down or stops; the general effect on the sphincters of the body; constriction of blood vessels in many parts of the body; liberation of nutrients (particularly fat and glucose) for muscular action; dilation of blood vessels for muscles; inhibition of the lacrimal gland (responsible for tear production) and salivation; dilation of pupil (mydriasis); relaxation of bladder; inhibition of erection; auditory exclusion (loss of hearing); tunnel vision (loss of peripheral vision); disinhibition of spinal reflexes; and shaking. Stress can cause or influence the course of many medical conditions including psychological conditions such as depression and anxiety. Medical problems can include fatigue, poor healing, irritable bowel syndrome, high blood pressure, poorly controlled diabetes and many other conditions.

The terms "sub-set of features" represents an exemplary embodiment of a combination of features (resulting from the combination of the first set of features step a) and the second set of features of step b)) which may allow the determination of a specific type of fatigue or fatigue related indicators selected among physical fatigue, mental fatigue, fatigue related to lack of oxygen, fatigue related to sleep troubles, or fatigue related to stress in a subject. In an exemplary embodiment, an optimal set of features corresponding to a specific type of fatigue or fatigue related indicators may be obtained, whereas in other exemplary embodiments there may be other combinations that work but are less effective.

In mathematics or statistics, a "combination" is a way of selecting items from a collection, such that (unlike permutations) the order of selection does not matter. A combination is a selection of all or part of a set of objects or features, without regard to the order in which objects or features are selected.

The "mean" is the average of the numbers, a calculated "central" value of a set of numbers. The first and the last half points are the first and the last points on the curve of the pulse wave having values equal to half of the values of the systolic peak amplitude, respectively.

As used in the present disclosure, "variation around the mean" is meant as including skewness, variance, entropy and standard deviation as defined below.

In probability theory and statistics, "skewness" is a measure of the asymmetry of the probability distribution of a real-valued random variable about its mean. The skewness value can be positive or negative, or even undefined.

"Variance" is a measurement of the spread between numbers in a data set. The variance measures how far each number in the set is from the mean. Variance is calculated by taking the differences between each number in the set and the mean, squaring the differences (to make them positive) and dividing the sum of the squares by the number of values in the set.

"Entropy" is a measure of randomness. Entropy is used to help model and represent the degree of uncertainty.

The "standard deviation" is a measure of the spread of scores within a set of data. By "derivatives of waveforms" it is meant that the first derivative is the velocity of the curve and the second derivative shows the acceleration or how fast the velocity of the curve changes.

The "ratio" means the division of two or more features or any function of features, and also includes the subtraction of at least two features and any function of features.

In an exemplary embodiment, a method may be provided of calculating, quantifying or determining the level of fatigue or fatigue related indicators selected among physical fatigue, mental fatigue, fatigue related to lack of oxygen, fatigue related to sleep troubles, fatigue related to stress or a combination thereof, in a subject having previously undergone a set of pulse wave recordation, said method including the steps of:

a) extracting and selecting from each single pulse wave and from its first and second derivation a first set of features selected among time, amplitude, area, ratios, heart rate and breathing rate;
b) performing a statistical analysis on said first set of features obtained from at least two pulse waves to arrive at a second set of features selected among mean, variation around mean and randomness between said first set of features of the at least two pulse waves and;
c) combining said first and second set of features and applying means configured in a software to analyze, determine and display results of fatigue or fatigue related indicators of said subject.

The software calculates the pre-selected combination of features after the preprocessing step involving the selection of convenient or good pulse waves and then applies it to the model programmed in the software to determine the level of fatigue or fatigue related indicators. The "pre-processing step" is the software development necessary prior to having a software program ready and in completed form to process collected pulse waves and apply selected features and algorithms to the data to estimate levels of fatigue and fatigue related indications. The algorithms developed upon the selection of optimal pulse wave features are integrated into software so that the software can then go through the necessary calculations and display the results in a set of visuals as shown by way of example in FIG 2. The pre-processing or software development includes programming that has the ability to take into consideration in the calculates various specific attributes of each individual such as age, gender, health conditions and other factors that might have an effect on the overall determination of fatigue and fatigue related indications.

According to an exemplary embodiment, pulse waves may have been collected and recorded beforehand, namely before carrying out the steps of the method. It is therefore noted that, according to such an embodiment, no diagnostic method involving the presence of a medical doctor or the subject (patient) is performed by performing all of the steps of the method. According to a preferred embodiment, the first set of features in step a) may be determined by measuring the entire pulse wave timeline, or by identifying a set of pulse wave points. In an embodiment, points may be selected from the following points: the systolic peak, diastolic peak, dicrotic notch, the first and last points corresponding to the half-height of the systolic peak, and the starting and ending points of said single pulse wave.

Preferably, the ratios in said first set of features of step a) may include the following:

The ratio of the amplitude of the systolic peak and the amplitude of the diastolic peak,
The ratio of the amplitude of the systolic peak and the amplitude of the dicrotic notch,
The ratio of the amplitude of the dicrotic notch and the amplitude of the diastolic peak,
The ratio of the time to reach the systolic peak and the time to reach the diastolic peak,
The ratio of the time to reach the systolic peak and the time to reach the dicrotic notch,
The ratio of the time to reach the dicrotic notch and the time to reach the diastolic peak,
The time difference between the time to reach the systolic peak and the time to reach the diastolic peak,
The time difference between the time to reach the systolic peak and the time to reach the dicrotic notch,
The time difference between the time to reach the dicrotic notch and the time to reach the diastolic peak,
The local cardiac output corresponding to the ratio of area under the curve to the time difference between the starting and ending time,
The local systolic cardiac output corresponding to the ratio of area under the curve between the starting point and the dicrotic notch by the time to reach the dicrotic notch,
The local diastolic cardiac output corresponding to the ratio of the area under the curve between the dicrotic notch and the ending point by the time difference between the time of the dicrotic notch and the time of the ending point,
The pulse width corresponding to the time difference between the first and the last points corresponding to the half-height of the systolic peak,
The pulse interval corresponding to the time difference between the ending and starting time,
The slope of the systolic cycle corresponding to the ratio of amplitude of the systolic peak to the time to reach the systolic peak,
The slope of the diastolic cycle corresponding to the ratio of the amplitude of the diastolic peak to the time difference between the ending point and the diastolic peak,
The diastolic decay corresponding to the logarithm of the slope of the diastolic cycle,
Inflection point area ratio corresponding to the ratio of area under the curve between the dicrotic notch and the ending point divided by the area under the curve between the starting point and the dicrotic notch,
The augmentation index as the ratio of the amplitude of the systolic peak divided by the amplitude of the diastolic peak, the ratio of the local diastolic cardiac output to the local systolic cardiac output, or the inverses thereof.

In particular, the variation around the mean in said second set of features of step b) may include skewness, variance and standard deviation.
Preferably, the randomness in said second set of features of step b) may include entropy.

According to a preferred embodiment, the means configured to analyse, determine and display results of fatigue or fatigue related indicators of said subject may include a software configured to calculate fatigue or fatigue related indicators in a predetermined and recommended manner. According to an exemplary embodiment, the software is configured to calculate a pre-selected combination of said first and second set of features after a preprocessing step involving the selection of convenient (or good) pulse waves and then to apply it to a model programmed in said software to determine the level of fatigue or fatigue related indicators.
In particular, the software may be configured to select an optimal sub-set of features resulting from the combination of said first and said second set of features of step c) through modelling as a sparse regularized optimization and applying greedy mathematical algorithms in order to characterize at least one of fatigue or a given fatigue related indicator.

According to another preferred embodiment, the set of pulse wave recordation may be collected during sleep of the subject. For example, a collection of pulse waves may be recorded at night when the subject is sleeping.

According to an exemplary embodiment, physical fatigue may at least include overload, performance, differentiation between overreach and non-overreach in sports activity and differentiation between well-recovered and non-recovered states in sports activity.

It has been surprisingly found that overload may be determined by an optimal sub-set of features including at least the combination of: variance of the time of the first point corresponding to the half-height of the systolic peak, skewness of the systolic peak amplitude, mean of the ratio of the amplitude of the systolic and amplitude of the dicrotic notch, and entropy of the ratio of amplitude of the systolic and the amplitude of the dicrotic notch.

In an exemplary embodiment, performance may be determined by an optimal sub-set of features including at least the combination of: variance of the diastolic decay, variance of the first half point time, variance of the inverse of the diastolic time, and the skewness of the diastolic time. In some exemplary embodiments, differentiation between overreach and non-overreach in sports activity may also be possible. This differentiation may be determined by an optimal sub-set of features including at least the combination of: the variance of diastolic decay and either the mean of diastolic decay or the variance of the pulse width.

In some exemplary embodiments, differentiation between well-recovered and not recovered states in sports activity may also be possible. This differentiation may be determined by an optimal sub-set of features including at least the combination of: the skewness of the inflection point area ratio and the skewness of pulse intervals.

According to an exemplary embodiment, fatigue related to sleep troubles may at least be associated with somnolence, sleep deprivation, and sleep efficiency. A method of calculating and determining the level of fatigue or fatigue related indicators may be directed at fatigue related to sleep troubles, which may at least include somnolence, sleep deprivation, sleep efficiency and lack of deep sleep and/or REM.

In some exemplary embodiments, sleep efficiency may be determined by a sub-set of features including at least the combination of: variance of the inverse of the diastolic time, variance of the inverse of the time difference between the systolic peak and the diastolic peak, skewness of the time of the first point corresponding to the half-height of the systolic peak, skewness of the breathing rate and the mean of the heart rate.

In some exemplary embodiments, differentiation between the levels of each of physical fatigue and fatigue related to sleep troubles may also be possible. Said differentiation may be determined by an optimal sub-set of features including at least the combination of: the skewness of the inverse of the time between the systolic peak and the diastolic peak and the skewness of the time of the first point corresponding to the half-height of the systolic peak.

It is known that blood pressure changes include changes in systolic blood pressure and diastolic blood pressure. In some exemplary embodiments, systolic blood pressure change may be determined by an optimal sub-set of features including at least the combination of: breathing rate entropy, skewness of the systolic peak amplitude, skewness of the inverse of diastolic time, skewness of the time difference between systolic and diastolic peak, skewness of the time difference between the dicrotic notch and the diastolic peak, skewness of the diastolic pulse area normalized by time, and skewness of diastolic decay as shown in Fig. 13, whereas diastolic blood pressure change may be determined by an optimal sub-set of features including at least the combination of: the ratio of the local diastolic cardiac output to the local systolic cardiac output, variance of the time difference between the dicrotic notch and the diastolic peak, skewness of the time difference between the dicrotic notch and the diastolic peak, skewness of the time ratio between the dicrotic notch and the diastolic peak, skewness of the time of the last point corresponding to the half-height of the systolic peak, skewness of diastolic decay, and the skewness of breathing rate as shown in Fig. 14.

The pulse wave (PW) is a complex physiological phenomenon observed and detected in blood circulation. A variety of factors may influence the characteristics of the PW, including arterial blood pressure, the speed and intensity of cardiac contractions, and the elasticity, tone and size of the arteries. The circulation of blood through the vascular system is also influenced by respiration, the autonomic nervous system and by other factors, which are also manifested in changes in the levels of fatigue. The cardiac function as seen in the PW alters in the presence of changes in levels of fatigue or fatigue related indicators. There are cardiovascular manifestations of fatigue even in healthy individuals.

Many of the features needed to analyze the PW for indications of levels of fatigue or fatigue related indicators can be taken by observing the contour of PWs over time. The typical PW shape is shown in FIG 4. Generally, there are two main components of the PW in the time domain: the forward moving wave and a reflected wave. The forward wave is generated when the heart (ventricles) contracts during systole. The reflected wave usually returns in the diastolic phase, after the closure of the aorta valves. The returned wave helps in the perfusion of the heart through the coronary vessels as it pushes the blood through the coronaries.

As noted in FIG 6, 40 features can be identified and observed in this diagram. As a starting point, there is feature extraction taken directly from a point based analysis of the PW timeline, which can provide seven PW points (that is, the five points specifically labeled in FIG. 4, as well as the start and end points). From these seven PW points, a group of features including amplitude, time, area and ratio may be derived. These may be referred to as the time and amplitude features where time denotes the distances between points on the PW and amplitude is the heights of the points calculated by measuring the distance between the lowest and highest points. There is also area based features, where areas under various PW points are calculated and used to obtain additional PW features. Similarly, different areas under the same waveform can be compared in the form of ratios or other forms of statistical analysis. Ratios are also determined by dividing these features among themselves.

Besides the timeline basis of feature selection, the frequency domain is also a way of obtaining additional PW features. The Fourier transform among other methods transfers the signal from time domain to frequency domain, which shows how much of the signal lies within each given frequency band over a range of frequencies. By comparing the original waveform and the transform data, some special features can be detected in the frequency domain. The breathing rate, one of the features that is a part of the groups of selected features described previously, may be obtained from the frequency domain as the breathing rate is captured at a lower frequency than the PPG frequency. The heart rate can also be obtained by this methodology. For a further selection of features may be useful to differentiate between sources of fatigue, it is also helpful to evaluate the derivatives of waveforms. The first derivative of a PW leads to its local velocity (velocity pulse wave). In order to compute it, one can approximate it by a finite difference operator. This allows the precise analysis of sudden changes in the waveform and the identification of features, which may not appear on a timeline basis. The second-order derivative which is the derivative of the first derivative (acceleration pulse wave) is helpful in obtaining additional features for indications of fatigue levels especially in cases where the timeline features are difficult to obtain as depicted in Fig. 5. All those collected features defined above are referred herein as the first set of features.

It is also helpful to use not only the features from the single PW (namely the first set of features) using these techniques but to also use the selected features in other statistical ways. For example, it may be of interest to see how the features change or evolve over time using, for example, additional parameters selected among mean, variation around mean and randomness and preferably selected among variability, variance, mean, standard deviations, entropy and skewness as noted in FIG 7 as a third main step of obtaining additional parameters needed to estimate levels of fatigue and fatigue related indicators, referred herein as the second set of features.

It is therefore helpful to have collected at least two PWs and preferably several PWs (i.e. tens, hundreds or thousands thereof) over an extended duration to allow such comparisons. Through these statistical analysis, the behavior or patterns of change in features even ratios of changes not just absolute values or averages of specific features of the PW are analyzed: variances, which is a mathematical calculation of how spread out PWs points are from their mean; skewness is a way of quantifying the extend which a distribution of PW features differs from a normal distribution. An exemplary embodiment of the method may also include another statistical analytic method of obtaining PW features, which is entropy as an appropriate measure of randomness.

From these statistical analytic methods used on the PW features identified on the PW timeline, an exemplary embodiment of the method can extract and identify at least 160 features as noted in FIG 7. This is done by using time, amplitude, area, and ratio to these PW features as identified in FIG 6. Several additional features are identified including breathing rate and heart rate, which is the time between each pulse wave. Further, as illustrated in FIG 7, all these features may then be used to statistically calculate their additional parameters selected among mean, variance, skewness and entropy to bring the total features used to 160 or more.

An exemplary embodiment of the method may also include a way of removing those features that have little or no correlation to changes in various forms of fatigue or fatigue related indicators. The F-test or similar mathematical solutions using anova solutions are a means of narrowing down the number of features. An "F-test" is any statistical test in which the test statistic has an F-distribution under the null hypothesis. It is most often used when comparing statistical models that have been fitted to a data set, in order to identify the model that best fits the population from which the data were sampled.

Through these statistical methods, the initial number of features can be reduced to around 70 features.

Since there may be some synergies between different PW features, an exemplary embodiment of a method may use a combination of features to identify correlations with various forms of fatigue or fatigue related indicators. Sparse mathematical methods are used to identify groups of features usually of no more than 7 features in each group. As illustrated in FIG 9, the sparse technique or related technique is used to obtain around 20 groups of features. Through greedy or related mathematical techniques also illustrated in FIG 9, each individual feature in each of these groups may be replaced one by one to identify the best or most indicative combination of features, which may be referred to herein as the optimal sub-set of features. These steps are repeated a few times until an optimal sub-set of features are identified. From this optimal sub-set or combination of features, algorithm(s) can be constructed either on a linear or nonlinear basis.

In an exemplary embodiment, pulse waves may be recorded beforehand. However the pulse wave diagnostic device according to an exemplary embodiment of a method can also collect blood pulse wave data for a period of time. Recordation of PWs can include several single pulse waves as shown in Fig 3; according to the exemplary embodiment of Fig. 3, the raw data is sent to the software. The software first decomposes it into a set of single pulse waves by finding local minimum points of the main wave. After a quality check, good pulses or convenient pulses are selected. A "convenient or good pulse wave" is defined as the one that has a shape of a reasonable blood pulse and one can identify systolic and diastolic peaks plus the dicrotic notch point.

A single pulse wave may be denoted by $p(t)$ where $t$ presents the time coordinate. Then, the collected pulse is $p_k = p(k \Delta t)$ where $\Delta t$ denotes the sampling step with k = 0,1, ,2,..., n. For example, assuming the subject heart rate is 60 beats/min, and the sampling rate of the device is 50 Hz then, in this example $n = 50$, and $\Delta t = 20$ *ms.* Note that first and second derivative of the pulse may be derived by using the finite difference method.

The pulse may be represented with a feature vector $f = [f_1, f_2,..., f_N]$ where $N$ is the number of features. In order to extract the characteristic feature vector for a single pulse, the following steps may be applied:

- First, systolic, diastolic peak and dicrotic notch may be determined, plus the first and the last half points as shown in Fig 4. The systolic peak is the first peak of the pulse (straightforward to find). The diastolic peak is the second one that can be more challenging to identify for some subjects (mostly for aged persons). If needed, in some exemplary embodiments, the first and the second derivative of the wave may be used to identify this point as illustrated in Fig 5. The dicrotic notch may be the local minima point of the signal. This may also be identified using the first and the second derivatives of the signal as depicted in Fig 5.

- The time and amplitude values of the later discovered key points may be calculated. These may use the following notations: aSystolic, aDiastolic, aDicrotic, tSystolic, tDiastolic, tDicrotic (for amplitudes and times respectively).

- The area under the curve is also computed by adding up a sampled points value multiplied by the sampling step. It is denoted by pulseArea.

- The area under the curve is also divided into two areas, which may be distinguished by the dicrotic notch point. The first one is between the starting point and the dicrotic notch, which is called the systolic area under the curve, and the area under curve between the dicrotic notch and the ending point of the signal, which may be called the diastolic area under the curve. They are denoted by areaSystolic and areaDiastolic, respectively.

- Normalizing the aforementioned area under the curves by the time period over which each one is calculated may yield the local cardiac output, which may in turn yield pulseAreatimeRatioSystolic, pulseAreatimeRatioDiastolic and pulseAreatime.

- The time interval between the starting and the ending points may be called the pulse interval and denoted by pulseInterval.

- The time interval between the first and the last half points may be called the pulse width and denoted by pulseWidth.

- The time difference may be calculated between each two of the systolic peak, the diastolic peak and the dicrotic notch.

- The time ratio may be calculated between each two of the systolic peak, the diastolic peak and the dicrotic notch.

- The amplitude ratio may be calculated between each two of the systolic peak, the diastolic peak and the dicrotic notch.

- The ratio of the areas may be calculated between the systolic area and the diastolic area.

In summary, first, the seven key points are identified (the systolic peak, the diastolic peak, the dicrotic notch, the first and last half and the ending and starting points). Then time, amplitude, and area linked to these points are computed. Then a generalized ratio may be defined, as shown in Fig 7, which computes the ratio and the difference of two features and inverse of a given value. An example is shown in the ratio of the amplitude of the systolic and diastolic points, and of the time difference between systolic and diastolic points, as shown in Fig 6.

It is important to note that these characteristic features are complementary. To illustrate this, the correlation between each two features may be calculated by considering a data-set of blood pulse waves which includes 100,000 single pulses. Figure 8 demonstrates the correlation image. The grayscale value is proportional to the correlation between the feature which corresponds to the row number and the feature which corresponds to the column number. In an exemplary embodiment, this may allow one to distinguish between different types of fatigues and can estimate fatigue and fatigue related indicators by using only blood pulse waves.

After extracting the characteristic features also referred herein as the first set of features for each pulse in a blood pulse wave, they may then be analyzed statistically by computing mean, variance, skewness and entropy for each feature over at least two ones as depicted in Fig 7. These features are referred as statistical features. In some exemplary embodiments, characteristic and statistical features may be used and combined to distinguish and determine fatigue and fatigue related factors. Then, it is necessary to select an optimal combination of features referred herein as optimal sub-set of features and to determine an optimal model to compute fatigue and fatigue related indicators using the selected combination.

According to an exemplary embodiment, a model may be applied, $\mathcal{M}: X \longrightarrow Y$, where $x \in X$ is a pulse wave feature vector and $y \in Y$ is fatigue or a fatigue related indicator. An optimal subset of features and an optimal model may be found by minimizing the loss function $\mathcal{L}(\mathcal{M}(x, a), y)$. The loss function measures how perfectly a model and a selected subset determine fatigue or a given fatigue indicator. Upon the identification of this optimal model, the optimal model is then integrated into the software. After preprocessing the collected pulse waves and filtering out the good quality ones using the software, an important step in the software is to use the model to evaluate levels of fatigue or a given fatigue related indicator. With this evaluation, the software is able to provide a form of visuals included in the software so that the users are able to observe in a user-friendly manner their respective levels of fatigue or fatigue related indicators. Because of the computational aspects of the model, the software may be located on a larger computational device such as cell phones or computers or the clouds. One can find the optimal sub-set of features and the optimal model using a brute force approach. This is a straightforward technique that goes through all possible sub-sets and finally selects the optimal one. As described, it requires high computation power. For example, in this case, it may be desired to find the optimal sub-set of features to estimate fatigue or fatigue related indicators. It is necessary to find what the number of features is and which features they are. In order to use a brute force approach, it may be necessary to search through all different possible combinations of features. If there are more than one hundred features, then the solution space has more than $10^{14}$ elements. Therefore, it requires a significant amount of time to go through all sub-sets, find the model, and compute the loss function value for each one. Moreover, because of the complicated nature of the clinical study to collect data, typically there is not a large amount of data and as a result there is a high risk of overfitting.

In order to overcome these issues, the problem may instead be formulated as a regularized optimization one:

$$\mathcal{J}(\mathcal{M}, a) = \operatorname{argmin} \{\mathcal{L}(\mathcal{M}, a, y) + \mathcal{R}(a)\},$$

where $\mathcal{L}$ is the loss function that measures how well fit the model $M$ to the measurement $y$, and the regularization term $\mathcal{R}$ includes the side information of the model for avoiding over-fitting. A first step of the framework has thus been determined. This may be demonstrated by a specific example:

- The model $\mathcal{M}$ may be assumed to be linear; it can be written in the form of:

$$\mathcal{M}(F) = F a$$

Where $a$ denotes a coefficient vector to describe the linear model $\mathcal{M}$ and each row of the matrix F is a pulse wave feature vector.

- The loss function is a least square error, $\mathcal{L}(F, y) = \| F a - y \|^2$, where $\|.\|^2$ is the $\ell_2$-norm.

- Sparsity regularization may be introduced by admitting $\mathcal{R}(a) = \| a \|_1$ where $\|a\|_1 = \Sigma_i |a_i|$ with $a_i$ is the i-th entry of the vector $a$.

- A fast iterative shrinkage thresholding algorithm may be used to solve the later equation. The absolute value of the coefficient vector $a$ may then be sorted. $K$ features with the maximum absolute coefficient values may be selected. This step may be repeated for different regularization parameters, and the set which results in the least value of the least-square error $\|F a - y\|^2$ may be selected. After fitting the optimal linear model to the selected set of features, and after selecting an optimal combination of features from a sparsity point of view, greedy algorithms may be used in order to find an optimal solution, namely the optimal sub-set, but close to the sparse solution. Closeness from this point of view means to have the maximum intersection with the sparse solution.

A "greedy algorithm" is an algorithm paradigm to find the global optimum by finding a local one in each step. In the present example, a user may be looking for an optimal set of features with size seven to estimate the fatigue or fatigue related indicators. They may start with an initial set which is the solution of the sparse representation. In each iteration, they may search for a group of local optimums such that new combinations differ with the last ones only in one feature (for example, 20 groups of feature combinations with seven features). This step maybe continued up to the convergence criteria. Therefore, the advantage of a greedy algorithm is converging in a reasonable number of iterations prior to finding optimal groups; typically, finding the optimal solution requires many number of iterations using brute force techniques. But, it can converge to local optimums instead and the solution in this case may depend on the initialization. Initializing with the solution of the sparse representation leads to the optimal combinations and guarantees not facing over-fitting by choosing the minimum number of features.

In summary, the steps of selecting and making available to the users an optimum sub-set of features and optimal model for identifying fatigue or a given fatigue related indicator involve:

1. Using regularized optimization and sparse representation to select an optimal combination of features with the minimum size.
2. Using greedy algorithms initializing with the feature combination of the last step to select better combinations with the same number of features.
3. The selected subset of features combined with the optimal model is integrated into the software. After pulse wave collection, the software is then able to calculate fatigue or a given fatigue related indicators using the optimal model and the optimal subset of features together with the pulse wave preprocessing step. The outcome of the software is then visualized in the form of a display or a set of numerical values.

One can improve the efficiency and the performance of the feature selection step by using F-test or anova to discard non-relevant features and then apply the aforementioned steps as shown in Fig 9. After selecting the optimal features, one can use a different learning approach for the final decision steps (finding the model). One simple model, which can be used in one exemplary embodiment, can be a linear model. Other examples, which may be used in other exemplary embodiments, include an artificial neural network, support vector machine, non-linear and polynomial models.

With the optimal group of features identified and an algorithm(s) designed to best use this group of features, the mathematical model can be built into the software used to identify levels of fatigue or fatigue related indicators. In some exemplary embodiments, these calculations can be contained in the software located on a device such as a mobile phone or computer, or can be in a cloud form, which, in turn, may be available to the user for example on the user's pulse wave diagnostic device.

In another exemplary embodiment, a pulse wave diagnostic device for determining and quantifying the level of fatigue or fatigue related indicators selected among physical fatigue, mental fatigue, fatigue related to lack of oxygen, fatigue related to sleep troubles or a combination thereof, in a subject may be provided. A pulse wave diagnostic device may be applied on a pulse-taking location on the body of said subject. Said pulse wave diagnostic device may include:

- means of extracting and selecting from each single pulse wave and from its first and second derivation a first set of features selected among time, amplitude, area, ratios, heart rate and breathing rate;
- means for performing a statistical analysis on said first set of features obtained from at least two pulse waves to arrive at a second set of features selected among mean, variation around mean, and randomness between said first set of features of the at least two pulse waves, and;
- means for combining said first and second set of features and means for transmitting these signals/data to a software configured to analyse and display results of the level of fatigue or fatigue related indicators of said subject.

The software calculates the pre-selected combination of features after the preprocessing step involving the selection of convenient or good pulse waves and then applies it to the model programmed in the software to determine the level of fatigue or fatigue related indicators. According to an exemplary embodiment, the software is configured to calculate a pre-selected combination of said first and second set of features after a preprocessing step involving the selection of convenient (or good or clear or suitable) pulse waves and then to apply it to a model programmed in said software to determine the level of fatigue or fatigue related indicators.

In particular, the software may be configured to select an optimal sub-set of features resulting from the combination of said first and said second set of features of step c) through modelling as a sparse regularized optimization and applying greedy mathematical algorithms in order to characterize at least one of fatigue or a given fatigue related indicator.

As defined above, physical fatigue may at least include overload and performance, and may at least include differentiation between overreach and non-overreach in sports activity and differentiation between well-recovered and non-recovered states in sports activity.

Similarly, fatigue related to sleep troubles includes at least somnolence, sleep deprivation and sleep efficiency. Specifically, the method of calculating and determining the level of fatigue or fatigue related indicators may analyse fatigue related to sleep troubles including at least somnolence, sleep deprivation, sleep efficiency, levels of light, and lack of deep sleep and/or REM sleep.

In an exemplary embodiment, the pulse wave diagnostic device may be adapted for personal health care diagnosis.

According to an exemplary embodiment, the means of extracting pulse wave signal may be selected among pulse-taking sensors, photo or video imaging, optical emitters based on LEDS or a combination thereof.

In an exemplary embodiment, the pulse wave diagnostic device may be deprived of a filter that distorts the pulse wave shape.

Heart-generated pulse waves propagate along the skin arteries, locally increasing and decreasing in blood volume with each heartbeat. The dynamic blood volume changes in relation to the heart function, size and elasticity of blood vessels and various neural processes. Blood absorbs more light than the surrounding tissue. Therefore, a reduction in the amount of blood is detected as an increase in the intensity of the detected light and vice versa. Photoelectric Plethysmography (PPG), which measures the degree of light absorption in a tissue based on the change in this peripheral blood flow rate, is an optical method of measuring pulse waves. Currently, this is the most popular means of acquiring pulse wave data. Other means are also available and may increase in popularity in the future.

Also referred to as pulse oximeters, the PPG hardware consists primarily of the following main components: 1) a sensor or a set of sensors; 2) integrated circuits or printed circuit board for inter-connection of the components 3) the memory units for storing the received pulse wave data 4) the networking systems that enable the transmission of the collected data within and across to other computing devices such as mobile phones; and 5) the software applications and device firmware that enable the computing units to perform a myriad of tasks individually and collectively. There is also usually a power source such as a battery to provide the power to run the various units.

FIG 1 is a representation of an exemplary embodiment of a pulse wave diagnostic device. This PPG probe includes one or several infrared light-emitting diodes (LEDs) and/or green or other color LEDs and one or several photodetectors.

Many combinations of these two main components are possible to try to best obtain pulse wave signals for as many different human physiological factors as possible such as pigmentation in tissue, venous configuration, bone and other features than can vary from person to person and body location (wrist, finger, ear, arm, etc.). The light sources from the optical emitters are LEDS which illuminate the tissue and the photodiodes which are photodetectors used to measure the variations in light intensity associated with the changes in blood vessel blood volumes. The array of sensors is designed to allow multiple colors, wavelengths, light angles, and distances between sensors to best characterize and acquire the pulse waves. This array of sensors is connected through an electronic circuit board to the memory unit and battery. In this system, according to some exemplary embodiments, operational amplifiers may be used to amplify the signals, and high resolution analogue-to-digital converters may also be used. Bluetooth is used to send the data to a larger computing device such as a mobile phone. The device also includes a mini USB to permit manual transmissions of data.

A variation on this optical sensor pulse wave acquisition is using photo or video imaging, also referred to as video plethysmography. It is also possible to capture pulse waves by taking either photos or a series of photos, which may be of a contact type for short-distance measurements (for example, this may require a user to place their finger on a mobile phone camera to use the phone camera LED light) or may be of a non-contact type for longer-distance measurements. According to an exemplary embodiment, some form of hardware may be used to capture quality pulse wave signals regardless of whether they are obtained from optical sensor technologies as described or from photo or visual imagery and/or any other means of obtaining a clear pulse wave, preferably the raw signal, which may allow the different pulse wave features to be distinguished. Accurate and reliable presentation of the pulse waveform is of importance. Other methods of acquiring the pulse wave may be contemplated in other exemplary embodiments.

In some exemplary embodiments, software may allow for acquiring, collecting, analyzing and displaying the analysis and interpretation of the pulse wave data in a user-friendly manner. The pulse wave diagnostic device may have inbuilt firmware to ensure the smooth running of the components including the operation of the sensors and the handling and storage of acquired pulse wave data. The pulse wave diagnostic device may also permit the transfer of the acquitted data to a larger computer processing device such as a mobile phone or computer.

Once the data is correctly transferred to a desired computer platform such as a mobile phone, apps such as mobile apps allow for further computation and provide the user with a good user experience. This includes good visuals so the user can quickly understand their levels of fatigue without being experts in the field. In an exemplary embodiment, the data may be security protected to ensure privacy.

To assess fatigue levels, a necessary step in the pulse wave diagnostic device is to collect the pulse wave data using the described or similar biosensor device or any type of device that can collect and register pulse waves.

Generally, pulse waves can be obtained from many parts or pulse-taking location of the body where there is access to pulses (wrist, finger, arm, ear, head, etc.). In an exemplary embodiment, the sensor may be configured to fit snugly against the chosen part of the body to avoid gaps between the sensor and the tissue. Biosensors in ear buds have, for example, a considerably different shape from a wrist-based location, which is more of a 2-dimensional surface.

If light gets in between the sensor(s) and the skin this will distort the pulse wave signals from ambient light, ranging from direct sunlight to flickering room light.

Pulse taking locations vary in vascular structure, which affect rates of blood perfusion as lower perfusion correlates with lower blood flow signals. The pulse wave shapes need to be considered since they can be different depending on the location of data collection. The pulse should also be taken, for example in an area where the artery is less likely to move as well as in an area where other movements such as muscle, tendon and bone can, if possible, be minimized to avoid unnecessary noise artefacts.

It may further be noted that data collection may be better when taken lying down or sitting to avoid abrupt body movement; however, it may also be noted that this is not required. Movements will cause motion artefacts, which can distort the signal quality. The fewer the number of artefacts, the less that needs to be done to filter out the noisy elements in the signal. For example, according to an exemplary embodiment, pulse waves may be measured during the night when the subject is asleep. This limits light and motion artefacts and permits a long period of data acquisition without requiring behavioral changes on the part of the subject. Overnight data collection is also valuable in that the data captured reflects the physiological changes due to the day's activities. A longer sample period also permits more accurate data analysis since erroneous data can be discarded as there are plenty of other pulse wave samples to choose from. It is therefore helpful to have collected at least two PWs and preferably several PWs (i.e. tens, hundreds or thousands thereof) over an extended duration to allow good comparisons.

A longer data collection period also allows for pulse wave features to be analyzed in terms of variance and variability. Often pulse wave analysis relies on absolute pulse wave features based on averages and means or even through the comparisons of single pulse waves. Having a larger data base of pulse waves over an extended period allows the analysis of the changes in pulse wave features through such additional variables as variance, variability and skewness. This is also helpful when machine learning and other mathematical techniques are applied where generally larger databases are needed.

To derive indications of levels of fatigue or fatigue related indicators, according to an exemplary embodiment, a pulse wave analysis may be performed using the full contours and features present in in a pulse wave, preferably an unfiltered pulse wave. Many pulse wave acquiring devices as described above use filters that distort the pulse wave shape to highlight the heart rate peaks. This is because the main objective of the device is to measure heart rates and the derived HRV. Filters are also used to remove environmental effects and other disturbances, which can change the morphology of the pulse wave. It may instead be desired to use raw pulse wave data; this data can be acquired either directly without signal manipulation or by removing the filters from the acquired filtered PPG signals. Reverse filters can also be applied. The acquired signals need to be examined to ensure clear pulse wave contours are obtained (herein defined as convenient pulse waves). Bad or distorted PPG signals need to be either corrected or discarded. Since there are lots of pulse waves in a sample, according to an exemplary embodiment, this may be accomplished through a program that "de-bugs" the signals by taking the bad signals out from the good ones. This part of the sensor system includes "signal quality flags", generated via signal processing, to indicate the quality of the biometric data and to inform the program to exclude low quality and erroneous data.

With the optimal sub-set or group of features identified and an algorithm(s) designed to best use this group of features, a level of fatigue or fatigue-related indicator may be identified. In some exemplary embodiments, a mathematical model can be built into the software used to identify levels of fatigue or fatigue related indicators. These calculations can be contained in the software located on a device such as a mobile phone or computer or it can be in a cloud form, which, in turn, is available to the user on the user's pulse wave diagnostic device.

In an exemplary embodiment, it may be desired to display a clear visual in the form of, for example, a battery depicting the level of energy remaining in the battery, an image used currently to illustrate battery life in a smart phone or computer (see Figure 2). A variation on this visual is to indicate a numerical value in a range of, say, 1 to 10. For users of the pulse wave diagnostic device that seek more detailed information on their pulse, the device may include the ability to obtain data of considerably more detail such as more specific aspects of fatigue related indicators or a breakdown of the different sources of fatigue (physical, mental and sleep related) and its relation to overall fatigue. The values of specific features or combination of features may also be indicated. The device is designed to also provide data on how the calculations are derived as well as provide fatigue and fatigue related indications for other health related web sites.

Those skilled in the art will appreciate that the invention described herein is susceptible to variations and modifications other than those specifically described. It is to be understood that the invention includes all such variations and modifications without departing from the spirit or essential characteristics thereof. The invention also includes all of the steps, features, compositions and compounds referred to or indicated in this specification, individually or collectively, and any and all combinations or any two or more of said steps or features. The present disclosure is therefore to be considered as in all aspects illustrated and not restrictive, the scope of the invention being indicated by the appended Claims, and all changes which come within the meaning and range of equivalency are intended to be embraced therein. Various references are cited throughout this specification, each of which is incorporated herein by reference in its entirety.

The foregoing description will be more fully understood with reference to the following Examples. Such Examples, are, however, exemplary of methods of practicing the present invention and are not intended to limit the scope of the invention.

**EXAMPLES**

Example 1:

Fifteen subjects were tested over a period of 51 days. All the subjects were university students between the ages of 20 and 30 (25 plus or minus 5), 7 were female, 8 were male. They were all considered generally healthy. The clinical trial consisted of three periods: baseline, over load, where the subjects were asked to over extend themselves with physical training and recovery, where the subjects decreased their training to the same or similar levels as during baseline. The subjects' pulse waves were recorded overnight using a pulse wave (PW) extraction and recording device like the device depicted in FIG 1; 5 times during the 14- day baseline; 7 times during the 21- day overload training; and 4 times during the 15-day recovery. The subjects ran a 3-kilometer distance regularly during these phases and did extra physical workouts during the overload phase. The 3- kilometers run was supervised and timed. The times it took to run the 3 kilometers were recorded and used as an indication of sports performance. The subjects ran the 3 kilometers at the same time each day.

The recorded data from the PWs for each of the 15 subjects along with their performance data were downloaded into Matlab for analysis. Unfiltered raw PWs were used and signal quality was examined with bad signals or distorted PWs removed from the data base to ensure reliable results. The first question is the possibility of estimation of the performance run test using the collected pulse wave during the night before. To respond this, we use the F-test was used as a means of determining which PW features were correlated to the subjects' sports performance by testing and removing one by one those features that were not distinguishing. Roughly 50 features were removed, leaving roughly 70 features as potentially interesting candidates from the analysis of variance (anova math techniques).

In order to overcome overfitting problem, Applicants aim at finding a linear model with a minimum number of features (simplicity in the model with low number of data can reduce the risk of overfitting) such that estimate the 3k run test performance. In order to select an optimal set of features and find a right model, Applicants formulate the problem as a regularized optimization problem with sparsity constrain. Applicants solve the regularized optimization problem using fast iterative shrinkage algorithm, Applicants find the coefficient vector and then Applicants select among the features the ones whose coefficient values in the model are significantly large. The output of this step gives us the optimal subset of features with the model to estimate the performance of 3k run test.

Since it is possible that some synergy exists between features, Applicants continue by finding optimal group of features close to the outcome of the sparse solution (more close means more number of features in common) which groups or sub-set of features were the most discriminatory. To do so, Applicants use greedy algorithms. Means that, Applicants first find the optimal group of features with only one different features compared to the sparse solution (finding local optimum). Applicants then continue the same technique on the selected optimal group of features which are the outcome of the last step. This technique was repeated at least 5 times to obtain the best groups or sub-set of features. Through this process, the best most optimal sub-set of PW features and the optimal model were found. The optimal model is reflected into an implementable algorithm. Upon the identification of this optimal model, the optimal model and the algorithm are then integrated into the software. After preprocessing the collected pulse waves and filtering out the good quality ones using the software, an important step in the software is to use the model to evaluate the performance. With this evaluation, the software is able to provide a form of visuals included in the software so that the users are able to observe in a user-friendly manner their respective levels of performance as shown in Figure 2. Because of the computational aspects of the model, the software may be located on a larger computational device such as cell phones or computers or the clouds. Using this optimal set of selected features and the optimal model integrated in the software, a high correlation for the estimates of performance may be achieved, as shown in FIG 12.

After integrating the optimal set of features and the optimal model for performance estimation into the software, Applicants validated the model on another athlete. The athlete accepted to test the software during his normal life. The software collects his data over some nights during one month study. Each time (the day after the data collection) the athlete ran 3 kilometers. His performance records were 11:49.49, 13:30.17, 11:07.70, 11:09.24, 11:02.17 and 10:59.59 minutes. Equivalently, the performances of the athlete compared to his first day changed around 15.48%, -4.82%, -4.60%, -5.61%, and -5.98%, respectively.

On the other side, the software processed the collected data over nights, calculated the values of the optimal set of features (1- the entropy of the inverse of the time difference between the systolic and dicrotic, 2- the skewness of the diastolic time, 3- the variance of the invers of the diastolic time, 4- the skewness of the inverse of the diastolic time, 5- the skewness of the ratio of the amplitude of dicrotic and diastolic points, 6- the skewness of the time difference between dicrotic and diastolic, and 7- the skewness of the second point corresponding to the half point value of the systolic peak)) and estimated the applicant performance using the optimal model,

$$change\ of\ performance$$
$$= 0.068695 + 0.074251\ itDiffSysDicrentropy$$
$$- 0.019642\ tDiastolicskewness - 0.14817\ itDiastolicvar$$
$$- 0.016331\ itDiastolicskewness - 0.013195\ aRatioDicrDiasskewness$$
$$+ 0.0094649\ tDiffDicrDiasskewness$$
$$- 0.0048981\ secondHalfPointskewness.$$

The estimated values are 13.12%, -4.70%, -2.28%, -5.89%, and -5.86%, respectively. The r-squared value of the model on the validation data is around 0.70 which confirms the accuracy of the proposed model.

In summary, first, the software could perfectly distinguish the day that his performance significantly dropped (around 15 percent) from the days that his performance improved (the last three data). Second, the software estimated the values of the performance change using the collected pulse waves during the night with high accuracy level.

Table 1.

| | | | |
|---|---|---|---|
| 'tDiastolicvar' 'diastolicDecayvar' 'slopeSystolicskewness' 'tDiffDicrDiasskewness' 'itSystolicentropy' 'diastolicDecayentropy' 'aDicroticvar' | 'tRatioSysDicrvar' 'diastolicDecayvar' 'slopeSystolicskewness' 'tDiffDicrDiasskewness' 'itSystolicentropy' 'BRentropy' 'diastolicDecayentropy' | pulseAreatimeDiastolicRatiomean' 'diastolicDecayvar' 'slopeSystolicskewness' 'tDiffDicrDiasskewness' 'itSystolicentropy' 'diastolicDecayentropy' | 'tDiffSysDiasskewness' 'diastolicDecayvar' 'slopeSystolicskewness' 'tDiffDicrDiasskewness' 'itSystolicentropy' 'diastolicDecayentropy' 'tRatioSysDicrvar' |
| 'itDicroticmean' 'diastolicDecayvar' 'slopeSystolicskewness' 'tDiffDicrDiasskewness' 'itSystolicentropy' 'diastolicDecayentropy' 'tRatioSysDiasvar' | 'aDiastolicmean' 'diastolicDecayvar' 'slopeSystolicskewness' 'tDiffDicrDiasskewness' 'itSystolicentropy' 'diastolicDecayentropy' 'tRatioSysDiasvar' | 'tDiastolicmean' 'diastolicDecayvar' 'slopeSystolicskewness' 'tDiffDicrDiasskewness' 'itSystolicentropy' 'diastolicDecayentropy' 'aDicroticvar' | 'itSystolicvar' 'diastolicDecayvar' 'slopeSystolicskewness' 'tDiffDicrDiasskewness' 'itSystolicentropy' 'diastolicDecayentropy' 'tRatioSysDiasvar' |
| Altimevar' 'diastolicDecayvar' 'slopeSystolicskewness' 'tDiffDicrDiasskewness' 'itSystolicentropy' 'BRentropy' 'diastolicDecayentropy' | 'tDiffSysDicrmean' 'diastolicDecayvar' 'slopeSystolicskewness' 'tDiffDicrDiasskewness' 'itSystolicentropy' 'diastolicDecayentropy' 'tRatioSysDiasvar' | tSystolicvar' 'diastolicDecayvar' 'slopeSystolicskewness' 'tDiffDicrDiasskewness' 'itSystolicentropy' 'diastolicDecayentropy' 'tRatioSysDicrvar' | 'Alentropy' 'diastolicDecayvar' 'slopeSystolicskewness' 'tDiffDicrDiasskewness' 'itSystolicentropy' 'diastolicDecayentropy' 'tRatioSysDicrvar' |
| 'aSystolicmean' 'diastolicDecayvar' 'slopeSystolicskewness' 'tDiffDicrDiasskewness' 'itSystolicentropy' 'diastolicDecayentropy' 'tRatioSysDicrvar' | 'BRentropy' 'diastolicDecayvar' 'slopeSystolicskewness' 'tDiffDicrDiasskewness' 'itSystolicentropy' 'diastolicDecayentropy' 'tRatioSysDiasvar' | 'itDiffSysDicrmean' 'diastolicDecayvar' 'slopeSystolicskewness' 'tDiffDicrDiasskewness' 'itSystolicentropy' 'diastolicDecayentropy' 'tRatioSysDicrvar' | itDicroticmean' 'diastolicDecayvar' 'slopeSystolicskewness' 'tDifiDicrDiasskewness' 'itSystolicentropy' 'diastolicDecayentropy' 'tRatioSysDicrvar' |

Example 2:

Fifteen subjects were tested over a period of 51 days. All the subjects were university students between the ages of 25 plus or minus 5; 7 were female; 8 were male. They were all considered generally healthy. The clinical trial consisted of three periods: baseline, over load, where the subjects were asked to over extend themselves with physical training and recovery, where the subjects decreased their training to the same or similar levels as during baseline. The subjects' pulse waves were recorded overnight using a pulse wave (PW) extraction and recording device like the device depicted in FIG 1, 5 times during the 14- day baseline; 7 times during the 21-day overload training; and 4 times during the 15-day recovery. The subjects ran a 3-kilometer distance regularly during these phases and did extra physical workouts during the overload phase. Most of the days a TRIMP "training impulse" was calculated for each subject. TRIMP is a measurement commonly used among those active in sports as a way of measuring physical effort. Instead of simply calculating the amount of time spent on a sports effort, the time is weighed based on the heart rates during the times measured. More weight is given to periods of high heart rates and lower weights are applied to times of lower heart rate periods. The TRIMP calculated training load is also averaged for extended periods of training. TRIMP and adjusted or weighed TRIMP is a good indication of training load or physical exertion placed on the body.

The recorded data from the PWs for each of the 15 subjects along with the TRIMP data were downloaded into Matlab for analysis. Unfiltered raw PWs were used and signal quality was examined with bad signals or distorted PWs removed from the data base to ensure reliable results. The F-test was used as a means of determining which PW features were correlated to the subjects' training load or TRIMP by testing and removing one by one those features that were not significant. Roughly 50 features were removed, leaving roughly 70 features as potentially interesting candidates from the analysis of variance (anova math techniques). Since it is possible that some synergy exists between features, the remaining roughly 70 selected features were placed into roughly 20 groups to get combinations of features so as to obtain an optimum sub-set of roughly 7 features, each using the mathematical sparse techniques as shown in table 2.

Using greedy math techniques, each of the features in each group were replaced one by one to determine which groups of features were the most significant in determining TRIMP levels. This technique was repeated at least 5 times to obtain the best groups or combination of features. Through this process, the best most optimal group or sub-set of PW features and the optimal model were found. The optimal model is reflected into an implementable algorithm. Upon the identification of this optimal model, the optimal model and the algorithm are then integrated into the software. After preprocessing the collected pulse waves and filtering out the good quality ones using the software, an important step in the software is to use the model to evaluate the physical load (TRIMPs).

A similar integration into the software and verification is done on more subjects as described in Example 1.

With this evaluation, the software is able to provide a form of visuals included in the software so that the users are able to observe in a user-friendly manner their respective levels of physical load (TRIMPs) as shown in Figure 2. Because of the computational aspects of the model, the software may be located on a larger computational device such as cell phones or computers or the clouds.

Table 2.

| | | | |
|---|---|---|---|
| 'tDicroticentropy' 'BRmean' 'firstHalfPointentropy' 'pulseIntervalmean' 'secondHalfPointvar' 'itDiffSysDicrentropy' 'tRatioSysDicrvar' | 'pulseAreaDiastolicmean' 'tDicroticvar' 'BRmean' 'firstHalfPointentropy' 'pulseIntervalmean' 'pulseWidthvar' 'tRatioSysDicrvar' | 'tDicroticentropy' 'BRmean' 'firstHalfPointentropy' 'pulseIntervalmean' 'pulseWidthvar' 'itDiffSysDicrentropy' 'tRatioSysDicrvar' | 'aRatioSysDiasvar' 'tDicroticvar' 'BRmean' 'firstHalfPointentropy' 'pulseWidthvar' 'itDiffSysDicrentropy' 'tRatioSysDicrvar' |
| 'tDicroticentropy' 'BRmean' 'firstHalfPointentropy' 'pulseIntervalmean' 'secondHalfPointvar' 'itDiffSysDicrentropy' 'tRatioSysDicrvar' | 'pulseAreaSystolicmean' 'tDicroticvar' 'BRmean' 'firstHalfPointentropy' 'pulseIntervalmean' 'secondHalfPointvar' 'tRatioSysDicrvar' | 'pulseAreamean' 'tDicroticvar' 'BRmean' 'firstHalfPointentropy' 'pulseIntervalmean' 'pulseWidthvar' 'tRatioSysDicrvar' | 'aRatioSysDiasvar' 'tDicroticvar' 'BRmean' 'firstHalfPointentropy' 'pulseIntervalmean' 'pulseWidthvar' 'tRatioSysDicrvar' |
| 'slopeDiastolicmean' 'tDicroticvar' 'BRmean' 'firstHalfPointentropy' 'pulseIntervalmean' 'pulseWidthvar' 'tRatioSysDicrvar' | pulseAreatimeSystolicRa tiomean' 'tDicroticvar' 'BRmean' 'firstHalfPointentropy' 'pulseIntervalmean' 'pulseWidthvar' | 'aSystolicmean' 'tDicroticvar' 'BRmean' 'firstHalfPointentropy' 'pulseIntervalmean' 'secondHalfPointvar' 'tRatioSysDicrvar' | 'aDicroticmean' 'tDicroticvar' 'BRmean' 'firstHalfPointentropy' 'pulseIntervalmean' 'pulseWidthvar' 'tRatioSysDicrvar' |
| itDicrolicentropy' 'BRmean' 'firstHalfPointentropy' 'pulseIntervalmean' 'secondHalfPointvar' 'itDiffSysDicrentropy' 'tRatioSysDicrvar' | aRatioSysDiasentropy' 'tDicroticvar' 'BRmean' 'firstHalfPointentropy' 'pulseIntervalmean' 'pulseWidthvar' 'tRatioSysDicrvar' | pulseAreatimeRatiomean' 'tDicroticvar' 'BRmean' 'firstHalfPointentropy' 'pulseIntervalmean' 'pulseWidthvar' 'tRatioSysDicrvar' | 'slopeSystolicmean' 'tDicroticvar' 'BRmean' 'firstHalfPointentropy' 'pulseIntervalmean' 'pulseWidthvar' 'tRatioSysDicrvar' |
| itDiffSysDicrentropy' 'tDicroticvar' 'BRmean' 'firstHalfPointentropy' 'pulseIntervalmean' 'pulseWidthvar' 'tRatioSysDicrvar' | aDiastolicmean' 'tDicroticvar' 'BRmean' 'firstHalfPointentropy' 'pulseIntervalmean' 'pulseWidthvar' 'tRatioSysDicrvar' | 'itDiffSysDiasentropy' 'tDicroticvar' 'BRmean' 'firstHalfPointentropy' 'pulseIntervalmean' 'pulseWidthvar' 'tRatioSysDicrvar' | itDiffSysDiasentropy' 'tDicroticvar' 'BRmean' 'firstHalfPointentropy' 'pulseIntervalmean' 'secondHalfPointvar' 'tRatioSysDicrvar' |

The results are shown in FIG 11.

Example 3:

Fifteen subjects were tested over a period of 17 days. All the subjects were university students, between the ages of 20.4 to 23.8 (22.1 plus or minus 1.7), 8 were female, 7 were male. They were all considered generally healthy. The clinical trial consisted of three periods: baseline, sleep deprivation and recovery. The subjects were asked to sleep normally during the baseline and recovery phases. However, during the sleep deprivation phase they were asked to sleep 3 consecutive nights for 3-4 hours, each. The subjects' pulse waves were recorded overnight using a pulse wave (PW) extraction and recording device like the device depicted in FIG 1 for 3- nights during baseline, 3-nights during sleep deprivation and 2- times during recovery. In addition, the subjects wore PSG sleep analysis equipment for 1- night at the end of each of the 3- phases.

The recorded data from the PWs for each of the 15 subjects along with the data from the PSG equipment was analyzed after all the data was downloaded into Matlab. Unfiltered raw PWs were used and signal quality was examined with bad signals or distorted PWs removed from the data base to ensure reliable results. The F-test was used as a means of determining which PW features were correlated to the subjects' sleep quality by testing and removing one by one those features that were not significant. Roughly 50 features were removed, leaving roughly 70 features as potentially interesting candidates from the analysis of variance (anova). Since it is possible that some synergy exists between features, the remaining roughly 70 selected features were placed into roughly 20 groups of roughly 5 features, each using the mathematical sparse techniques as shown in Table 3.

Using greedy math techniques, each of the features in each group were replaced one by one to determine which groups of features were the most significant. This technique is repeated at least 5 times to obtain the best groups that is the best combination of features. Through this process, the best most optimal group or sub-set of PW features and the optimal model were found. The optimal model is reflected into an implementable algorithm. Upon the identification of this optimal model, the optimal model and the algorithm are then integrated into the software. After preprocessing the collected pulse waves and filtering out the good quality ones using the software, an important step in the software is to use the model to evaluate the sleep efficiency. With this evaluation, the software is able to provide a form of visuals included in the software so that the users are able to observe in a user-friendly manner their respective levels of sleep efficiency as shown in Figure 2. Because of the computational aspects of the model, the software may be located on a larger computational device such as cell phones or computers or the clouds.

Table 3.

| | | | | |
|---|---|---|---|---|
| 'aRatioSysDicrvar'<br>'pulseAreaSystolicvar'<br>diastolicDecayskewness'<br>'tSystolicvar'<br>'tRatioSysDiasvar' | 'pulseAreaSystolicvar'<br>'tSystolicvar'<br>diastolicDecayskewness'<br>'tRatioSysDiasvar'<br>'aRatioDicDiasmean' | 'itDicroticmean'<br>'Brvar'<br>'aDiastolicvar'<br>'slopeDiastolicvar'<br>'aRatioDicrDiasmean' | 'BRvar'<br>'diastolicDecayskewness'<br>'aDiastolicvar'<br>'slopeDiastolicvar'<br>'aRatioDicrDiasmean' | 'pulseAreaSystolicvar'<br>'diastolicDecayskewness'<br>'aDiastolicvar'<br>'slopeDiastolicvar'<br>'aRatioDicrDiasmean' |
| 'tDicroticmean'<br>'BRvar'<br>'aDiastolicvar'<br>'slopeDiastolicvar'<br>'aRatioDicrDiasmean' | 'tDiffSysDicrvar'<br>'pulseAreaSystolicvar'<br>'tSystolicvar'<br>'tRatioSysDiasvar'<br>'aRatioDicrDiasmean' | 'pulseAreatimeRatiovar'<br>'diastolicDecayskewness'<br>'tSystolicvar'<br>'tRatioSysDiasvar'<br>'aRatioDicrDiasmean' | 'BRvar'<br>'tRatioDicrDiasmean'<br>'aDiastolicvar'<br>'slopeDiastolicvar'<br>'aRatioDicrDiasmean' | 'tDiffDicrDiasmean'<br>'diastolicDecayskewness'<br>'aDiastolicvar'<br>'slopeDiastolicvar'<br>'aRatioDicrDiasmean' |
| 'tDiastolicskewness'<br>'BRvar'<br>'aDiastolicvar'<br>'slopeDiastolicvar'<br>'aRatioDicrDiasmean' | 'pulseAreatimeDiastolic<br>Ratiovar'<br>'BRvar'<br>'aDiastolicvar'<br>'slopeDiastolicvar' | slopeDiastolicvar'<br>'pulseAreaSystolicvar'<br>'diastolicDecayskewness'<br>'itDiffSysDiasskewness'<br>'aRatioDicrDiasmean' | tSystolicvar'<br>'BRvar'<br>'aDiastolicvar'<br>'slopeDiastolicvar'<br>'aRatioDicrDiasmean' | BRvar'<br>'firstHalfPointentropy'<br>'aDiastolicvar'<br>'slopeDiastolicvar'<br>'aRatioDicrDiasmean' |
| 'RatioDicrDiasmean'<br>'aDiastolicvar'<br>'slopeDiastolicvar'<br>'itDiffSysDiasskewness'<br>'aRatioDicrDiasmean' | slopeDiastolicvar'<br>'diastolicDecayskewness'<br>'aDiastolicvar'<br>'itDiffSysDiasskewness'<br>'aRatioDicrDiasmean' | BRvar'<br>'aDiastolicvar'<br>'slopeDiastolicvar'<br>'itDiffSysDiasskewness'<br>'aRatioDicrDiasmean' | tRatioDicrDiasmean'<br>'diastolicDecayskewness'<br>'aDiastolicvar'<br>'slopeDiastolicvar'<br>'aRatioDicrDiasmean' | tDiastolicskewness'<br>'aDiastolicvar'<br>'slopeDiastolicvar'<br>'itDiffSysDiasskewness'<br>'aRatioDicrDiasmean' |

Example 4:

In order to determine, from PW features, whether in sports training one is considered in overreach or non-overreach, fifteen subjects were tested over a period of 51 days. All the subjects were university students between the ages of 25 plus or minus 5; 7 were female; 8 were male. They were all considered generally healthy. The clinical trial consisted of three periods: baseline, over load, where the subjects were asked to over extend themselves with physical training and recovery, where the subjects decreased their training to the same or similar levels as during baseline. The subjects' pulse waves were recorded overnight using a pulse wave (PW) extraction and recording device like the device depicted in FIG 1; 5 times during the 14- day baseline; 7 times during the 21- day overload training; and 4 times during the 15-day recovery. The subjects ran a 3-kilometer distance regularly during these phases and did extra physical workouts during the overload phase. The 3- kilometers run was supervised and timed. The times it took to run the 3 kilometers were recorded and used as an indication of sports performance. The subjects ran the 3 kilometers at the same time each day.

In addition, each of the subjects were asked regularly to complete three questionnaires: an 80 question POMS questionnaire, an 8-item questionnaire and a third 14 item questionnaire. Heart Rate variability was also recorded on a regular basis.

The recorded data from the PWs for each of the 15 subjects along with their performance data and the questionnaire data and HRV data were downloaded into Matlab for analysis. Unfiltered raw PWs were used and signal quality was examined with bad signals or distorted PWs removed from the data base to ensure reliable results. The Coach categorized the groups into two groups: over-reach and non-over-reach. This is shown in FIG 10. The same methodology described previously may be applied to identify two most optimal features to distinguish between the two groups. This problem, selecting optimal group of features and finding the model to perfectly distinguish two groups, is tricky to solve. To do so, Applicants assign a numeric value to each group, the overreach group index 1 and the non-overreach group index -1. Then Applicants formulate the problem as a regularized optimization one with sparsity regularization. This is a non-linear optimization problem. Applicants use a fast-iterative shrinkage approach to solve it. Applicants select the features with large coefficient values in the solution vector. These two features are the variance of diastolic decay and the either of the mean of the diastolic decay or the variance of the pulse width. In addition, the final model based on these two features is:

$$output = sign(-.067343 pulseWidthvar - .0768 diastolicDecayvar + 1.3573),$$

where the output is 1 when the subject is over reach. The optimal model is reflected into an implementable algorithm. Upon the identification of this optimal model, the optimal model and the algorithm are then integrated into the software. After preprocessing the collected pulse waves and filtering out the good quality ones using the software, an important step in the software is to use the model to discriminate between overreach and non-overreach. With this evaluation, the software is able to provide a form of visuals included in the software so that the users are able to observe in a user-friendly manner whether he is in overreach stage. Because of the computational aspects of the model, the software may be located on a larger computational device such as cell phones or computers or the clouds.

Example 5:

In this example, the PW features may be identified, and an algorithm may be used to distinguish between physical related or source of fatigue and lack of sleep related or cause of fatigue. To arrive at this combination of extracted features, data from two different groups of subjects, each with 15 subjects, is used. Fifteen subjects were tested over a period of 51 days. All the subjects were university students between the ages of 25 plus or minus 5; 7 were female; 8 were male. They were all considered generally healthy. The clinical trial consisted of three periods: baseline, over load, where the subjects were asked to over extend themselves with physical training and recovery, where the subjects decreased their training to the same or similar levels as during baseline. The subjects' pulse waves were recorded overnight using a pulse wave (PW) extraction and recording device like the device depicted in FIG 1; 5 times during the 14- day baseline; 7 times during the 21- day overload training; and 4 times during the 15-day recovery. The subjects ran a 3-kilometer distance regularly during these phases and did extra physical workouts during the overload phase. The 3- kilometers run was supervised and timed. The times it took to run the 3 kilometers were recorded and used as an indication of sports performance. The subjects ran the 3 kilometers at the same time each day.

The second group of fifteen subjects were tested over a period of 17 days. All the subjects were university students between the ages of 22.1 plus minus 1.7; 8 were female; 7 were male. They were all considered generally healthy. The clinical trial consisted of three periods: baseline, sleep deprivation and recovery. The subjects were asked to sleep normally during the baseline and recovery phases. However, during the sleep deprivation phase they were asked to sleep 3 consecutive nights for 3-4 hours, each. The subjects' pulse waves were recorded overnight using a pulse wave (PW) extraction and recording device like the device depicted in FIG 1 for 3- nights during baseline, 3-nights during sleep deprivation and 2- times during recovery. In addition, the subjects wore PSG sleep analysis equipment for 1- night at the end of each of the 3- phases.

The last day of sleep deprivation may be examined, and all the features in the PW for all the 15 subjects for the last day of sleep deprivation, which is the third night of sleeping around 3 to 4 hours per night, may be identified. Similarly, all the PW features taken from the 15 subjects who have undergone the last day of over load in the physical training may be extracted. In one clinical study, there are 15 subjects which experiences a lack of sleep and in another clinical study there are 5 subjects which the coach distinguished as physically over-reached. (FIG 15). The same methodology previously described in the patent description may be used to obtain two features that are considered the most distinguishing between these two groups. These two features are the skewness of the first point corresponding to the half-height of the systolic peak and the skewness of the inverse of the time difference between the systolic and diastolic peak. From these two main features and algorithm is developed and the software as described in the other examples is then able to provide a set of visuals as depicted in FIG 2.

Example 6:

Currently the Doctor or therapist has no objective means of identifying the fatigue and determine the level of fatigue and compare it over periods of time. Usually, a doctor or therapist will typically interrogate the patient to try to find the cause of fatigue, which could include the use of questionnaires. The problem with these interrogative techniques is their reliability, effectiveness, subjectivity and ability to monitor progress. Questionnaires are considerably subjective and prone to considerable error. There is no way to tell how truthful the responses are, how much thought a respondent has put into it, nor if they interpret the questions in the right manner. Answers are influenced by moods and emotions at that time of the self-assessment and vary in the weights placed on their meaning. Fatigue is often a subjective feeling reported by the individual rather than an objective one. Fatigue and feelings of fatigue are often confused. For example, in Table 4 responses to a questionnaire on fatigue (with higher numbers corresponding to higher levels of feelings of fatigue) show little correlation to their performances in 3 kilometers runs measured in seconds. Table 4 below shows the results of a POMS questionnaire given to 5 subjects in Applicant's clinical trial. The three first columns are the recorded levels of fatigue as indicated in the answers given by the subjects during three phases of the study: baseline, overload and recovery. The next three columns are the time it took to run three kilometers (in seconds) for each of these same subjects during these three phases.

Table: 4

|  | POMS | | | Performance 3K run test | | |
|---|---|---|---|---|---|---|
|  | BSL | OVL | RCV | BSL | OVL | RCV |
| AAL90 | 16 | 7 | 8 | 601 | 628 | 593 |
| ADM72 | 3 | 8 | 1 | 743 | 762 | 732 |
| AJR92 | 2 | 15 | 1 | 678 | 694 | 655 |
| ASH96 | 4 | 8 | 1 | 780 | 785 | 763 |
| ALL93 | 6 | 15 | 9 | 627 | 596 | 583 |

ABBREVIATION LIST:

| Abbreviation | Definition |
|---|---|
| aSystolic | The amplitude of the systolic peak |
| aDiastolic | The amplitude of the diastolic peak |
| aDicrotic | The amplitude of the dicrotic notch |
| tSystolic | The time to reach the systolic peak |
| tDiastolic | The time to reach the diastolic peak |
| tDicrotic | The time to reach the dicrotic notch |

(continued)

| Abbreviation | Definition |
|---|---|
| pulseArea | Area under the curve |
| areaSystolic | Area under the curve between the starting point and dicrotic notch |
| areaDiastolic | Area under the curve between the dicrotic notch and the ending point |
| firstHalfPoint | Time of the first point corresponding to the half-height of the systolic amplitude |
| lastHalfPoint | Time of the last point corresponding to the half-height of the systolic amplitude |
| aRatioSysDias | Ratio of the amplitude of the systolic peak and the amplitude of the diastolic peak |
| aRatioSysDicr | Ratio of the amplitude of the systolic peak and the amplitude of the dicrotic notch |
| aRatioDicrDias | Ratio of the amplitude of the dicrotic notch and the amplitude of the diastolic peak |
| tRatioSysDias | Ratio of the time to reach the systolic peak and the time to reach the diastolic peak |
| tRatioSystDicr | Ratio of the time to reach the systolic peak and the time to reach the dicrotic notch |
| tRatioDicrDias | Ratio of the time to reach the dicrotic notch and the time to reach the diastolic peak |
| tDiffSysDias | Time difference between the time to reach the systolic peak and the time to reach the diastolic peak |
| tDiffSysDicr | Time difference between the time to reach the systolic peak and the time to reach the dicrotic notch |
| tDiffDicrDias | Time difference between the time to reach the dicrotic notch and the time to reach the diastolic peak |
| pulseAreatimeRatio | Local cardiac output corresponding to the ratio of the area under the curve to the time difference between the starting and ending time |
| pulseAreatimeSystolicRatio | Local systolic cardiac output corresponding to the ratio of the area under the curve between the starting point and the dicrotic notch to the time of the dicrotic notch |
| pulseAreatimeDiastolicRatio | Local diastolic cardiac output corresponding to the ratio of the area under the curve between the dicrotic notch and the ending point by the time difference between the time of the dicrotic notch and the time of the ending point |
| pulseWidth | Pulse width corresponding to the time difference between the first and the last points corresponding to the half-height of the systolic peak |
| pulseInterval | Pulse interval corresponding to the time difference between the ending and starting time |
| slopeSystolic | Slope of systolic corresponding to the ratio of the amplitude of the systolic peak to the time to reach the systolic peak |
| slopeDiastolic | Slope of diastolic corresponding to the ratio of the amplitude of the diastolic peak to the time difference between the ending point and the diastolic peak |
| diatolicDecay | Diastolic decay corresponding to the logarithm of the slope of the diastolic peak |
| IPA | Inflection point area ratio corresponding to the ratio of area under the curve between the dicrotic notch and the ending point divided by the area under the curve between the starting point and the dicrotic notch |
| AI | Augmentation index as the ratio of the amplitude of the systolic peak divided by the amplitude of the diastolic peak |
| IPAtime | Ratio of the local diastolic cardiac output by the local systolic cardiac output. |
| iX | The inverse of parameter X. For example, itSystolic is the inverse of systolic time |

**Claims**

1. A method of calculating and determining the level of fatigue or fatigue related indicators selected among physical fatigue, mental fatigue, fatigue related to lack of oxygen, fatigue related to sleep troubles, fatigue related to stress or a combination thereof, in a subject having previously undergone a set of pulse wave recordation, said method comprising the steps of:

   a) extracting and selecting from each single pulse wave and from its first and second derivation a first set of features selected among time, amplitude, area, ratios, heart rate and breathing rate;
   b) performing a statistical analysis on said first set of features obtained from at least two pulse waves to arrive at a second set of features selected among mean, variation around the mean, and randomness between said first set of features of the at least two pulse waves; and
   c) combining said first and second set of features and applying means configured in a software to analyse, determine and display results of fatigue or fatigue related indicators of said subject.

2. The method of calculating and determining the level of fatigue or fatigue related indicators according to claim 1, wherein the first set of features in step a) are determined by measuring the entire pulse wave timeline, or by identifying a set of pulse wave points selected among the systolic, diastolic, dicrotic notch, the first and last points corresponding to the half-height of the systolic peak and the starting and ending points of said single pulse wave.

3. The method of calculating and determining the level of fatigue or fatigue related indicators according to claim 1 or 2, wherein ratios in said first set of features of step a) comprises:

   A ratio of an amplitude of a systolic peak and an amplitude of a diastolic peak,
   A ratio of the amplitude of the systolic peak and an amplitude of a dicrotic notch,
   A ratio of the amplitude of the dicrotic notch and the amplitude of the diastolic peak,
   A ratio of a time value of the systolic peak and a time value of the diastolic peak,
   A ratio of the time value of the systolic peak and a time value of the dicrotic notch,
   A ratio of the time value of the dicrotic notch and the time value of the diastolic peak,
   A time difference between the time value of the systolic peak and the time value of the diastolic peak,
   A time difference between the time value of the systolic peak and the time value of the dicrotic notch,
   A time difference between the time value of the dicrotic notch and the time value of the diastolic peak,
   A local cardiac output corresponding to a ratio of an area under the curve to a time difference between a starting time and an ending time,
   A local systolic cardiac output corresponding to a ratio of an area under the curve between the starting point and the dicrotic notch to the time value of the dicrotic notch,
   A local diastolic cardiac output corresponding to a ratio of an area under the curve between the dicrotic notch and the ending point to the time difference between the time value of the dicrotic notch and the time value of the ending point,
   A pulse width corresponding to a time difference between the first and the last points corresponding to the half-height of the systolic peak,
   A pulse interval corresponding to the time difference between the ending and starting time,
   A slope of the systolic peak corresponding to the ratio of the amplitude of the systolic peak by the time value of the systolic peak,
   A slope of the diastolic peak corresponding to the ratio of the amplitude of the diastolic peak by the time difference between the ending point and the diastolic peak,
   A diastolic decay corresponding to a logarithm of the slope of the diastolic peak,
   A inflection point area ratio corresponding to the ratio of the area under the curve between the dicrotic notch and the ending point divided by the area under the curve between the starting point and the dicrotic notch,
   An augmentation index, corresponding to the ratio of the amplitude of the systolic peak divided by the amplitude of the diastolic peak,
   the ratio of the local diastolic cardiac output by the local systolic cardiac output,

   or the inverses thereof.

4. The method of calculating and determining the level of fatigue or fatigue related indicators according to any of claims 1 to 3, wherein said variation around the mean in said second set of features of step b) consists of skewness, variance and standard deviation.

5. The method of calculating and determining the level of fatigue or fatigue related indicators according to any of claims 1-4, wherein said randomness in said second set of features of step b) consists of entropy.

6. The method of calculating and determining the level of fatigue or fatigue related indicators according to any of claims 1 to 5, wherein the software is configured to calculate a pre-selected combination of said first and second set of features after a preprocessing step involving the selection of convenient pulse waves and then to apply it to a model programmed in said software to determine the level of fatigue or fatigue related indicators.

7. The method of calculating and determining the level of fatigue or fatigue related indicators according to claim 6, wherein the software is configured to select an optimal sub-set of features resulting from the combination of said first and said second set of features of step c) through modelling as a sparse regularized optimization and applying greedy mathematical algorithms in order to characterize at least one of fatigue or a given fatigue related indicator.

8. The method of calculating and determining the level of fatigue or fatigue related indicators according to any of claims 1 to 7, wherein the set of pulse wave recordation has been collected during sleep of the subject.

9. The method of calculating and determining the level of fatigue or fatigue related indicators according to any of claims 1 to 8, wherein physical fatigue comprises at least one of overload, performance, differentiation between overreach and non-overreach in sports activity and differentiation between a well-recovered state and a non-recovered state in sports activity.

10. The method of calculating and determining the level of fatigue or fatigue related indicators according to any of claims 1 to 8, wherein fatigue related to sleep troubles comprises at least one of somnolence, sleep deprivation, sleep efficiency, and lack of deep sleep and/or REM.

11. The method of calculating and determining the level of fatigue or fatigue related indicators according to claim 9, wherein overload is determined by an optimal sub-set of features comprising at least the combination of: a variance of the time of the first point corresponding to the half-height of the systolic peak, a skewness of the systolic peak amplitude, a mean of the ratio of the amplitude of the systolic peak and the amplitude of the dicrotic notch, and an entropy of the ratio of the amplitude of the systolic peak and the amplitude of the dicrotic notch.

12. The method of calculating and determining the level of fatigue or fatigue related indicators according to claim 9, wherein performance is determined by an optimal sub-set of features comprising at least the combination of: a variance of the diastolic decay, a variance of the first half point time, a variance of the inverse of the diastolic time, and the skewness of the diastolic time.

13. The method of calculating and determining the level of fatigue or fatigue related indicators according to claim 9, wherein the differentiation between overreach and non-overreach in sport activity is determined by an optimal sub-set of features comprising at least the combination of: the variance of diastolic decay and either the mean of diastolic decay or the variance of the pulse width.

14. The method of calculating and determining the level of fatigue or fatigue related indicators according to claim 9, wherein the differentiation between the well-recovered state and the non-recovered state in sports activity is determined by an optimal sub-set of features comprising at least the combination of: the skewness of inflection point area ratio and the skewness of pulse intervals.

15. The method of calculating and determining the level of fatigue or fatigue related indicators according to claim 10, wherein sleep efficiency is determined by an optimal sub-set of features comprising at least the combination of: a variance of the inverse of diastolic time, a variance of the inverse of the time difference between the systolic peak and the diastolic peak, skewness of the time of the first point corresponding to the half-height of the systolic peak, skewness of breathing rate and mean of heart rate.

16. The method of calculating and determining the level of fatigue or fatigue related indicators according to any of claims 1-10, wherein the differentiation and the levels of each of physical fatigue and fatigue related to sleep troubles are determined by an optimal sub-set of features comprising at least the combination of: the skewness of the inverse of the time between the systolic peak and the diastolic peak and the skewness of the time of the first point corresponding to the half-height of the systolic peak.

17. A pulse wave diagnostic device for determining and quantifying the level of fatigue or fatigue related indicators selected among physical fatigue, mental fatigue, fatigue related to lack of oxygen, fatigue related to sleep troubles or a combination thereof and fatigue related to stress, in a subject, said pulse wave diagnostic device being applied on a pulse-taking location on the body of said subject; said pulse wave diagnostic device comprising:

- means of extracting and selecting from each single pulse wave and from its first and second derivation a first set of features selected among time, amplitude, area, ratios, heart rate and breathing rate;
- means for performing a statistical analysis on said first set of features obtained from at least two pulse waves to arrive at a second set of features selected among mean, variation around the mean, and randomness between said first set of features of the at least two pulse waves; and
- means for combining said first and second set of features and means for transmitting these signals to a software configured to analyse and display results of the level of fatigue or fatigue related indicators of said subject.

18. The pulse wave diagnostic device for determining and quantifying the level of fatigue or fatigue related indicators according to claim 17, wherein physical fatigue comprises at least one of overload, performance, differentiation between overreach and non-overreach in sports activity and differentiation between a well-recovered state and a non-recovered state in sports activity.

19. The pulse wave diagnostic device for determining and quantifying the level of fatigue or fatigue related indicators according to claim 17, wherein fatigue related to sleep troubles comprises at least one of somnolence, sleep deprivation, sleep efficiency, and lack of deep sleep and/or REM.

20. The pulse wave diagnostic device for determining and quantifying the level of fatigue or fatigue related indicators according to any of claims 17-19, wherein said pulse wave diagnostic device is adapted for personal health care diagnosis.

21. The pulse wave diagnostic device for determining and quantifying the level of fatigue or fatigue related indicators according to any of claims 17-20, wherein said means of extracting pulse wave signal are selected among pulse-taking sensors, photo or video imaging, optical emitters based on LEDS, pulse oximeters, or a combination thereof.

22. The pulse wave diagnostic device for determining and quantifying the level of fatigue or fatigue related indicators according to any of claims 17-21, wherein said pulse wave diagnostic device is configured to provide an output without filtering the output and distorting the pulse wave shape.

FIG 1.

FIG 2

FIG 3.

Collected PPG

Determination of
local minimum
points

Decomposition into
its periods
(single pulse wave)

Preprocessing
and Quality check of
all pulses

Keep high quality
ones for analysis

FIG 4.

FIG 5.

FIG 6

EP 3 403 572 A1

PPG pulse wave

Characteristic feature extraction

Charcteristic features

Systolic peak, Diastolic peak, Dicrotic notch
starting and ending points
first and last half point

Amplitude, Area, Time

| | | | |
|---|---|---|---|
| 1-aSystolic | 4-tSystolic | 7-areaSystolic | 10-firstHalfPoint |
| 2-aDiastolic | 5-tDiastolic | 8-areaDiastolic | 11-lastHalfPoint |
| 3-aDicrotic | 6-tDicrotic | 9-pulseArea | |

Ratio

| | | |
|---|---|---|
| 12-aRatioSysDias | 21-areatimeSystolicRatio | 30-pulseWidth |
| 13-aRatioSysDicr | 22-areatimeDiastolicRatio | 31-pulseInterval |
| 14-aRatioDicrSys | 23-pulseAreatimeRatio | 32-DiastolicDecay |
| 15-tRatioSysDias | 24-itSystolic | 33-slopeSystolic |
| 16-tRatioSysDicr | 25-itDiastolic | 34-slopeDiastolic |
| 17-tRatioDicrSys | 26-itDicrotic | 35-AI |
| 18-tDiffSysDias | 27-itDiffSysDias | 36-AItime |
| 19-tDiffSysDicr | 28-itDiffSysDicr | 37-IPA |
| 20-tDiffDicrSys | 29-itDiffDicrSys | |

+

| | | |
|---|---|---|
| 38- HR | 39-BR | 40-rationAB |

FIG 7

EP 3 403 572 A1

FIG 8

'aSystolic'
'aDiastolic'
'aDicrotic'
'tSystolic'
'tDiastolic'
'tDicrotic'
'itSystolic'
'itDiastolic'
'itDicrotic'
'pulseInterval'
'aRatioSysDias'
'aRatioSysDicr'
'aRatioDicrDias'
'tDiffSysDias'
'tDiffSysDicr'
'tDiffDicrDias'
'itDiffSysDias'
'itDiffSysDicr'
'itDiffDicrDias'
'tRatioSysDias'
'tRatioSysDicr'
'tRatioDicrDias'
'slopeSystolic'
'slopeDiastolic'
'AI'
'pulseAreaSystolic'
'pulseAreaDiastolic'
'pulseArea'
'pulseAreatimeSystolicRatio'
'pulseAreatimeDiastolicRatio'
'pulseAreatimeRatio'
'AItime'
'IPA'
'firstHalfPoint'
'secondHalfPoint'
'pulseWidth'
'diastolicDecay'
'ratioAB'

FIG 9

FIG 10

FIG 11.

Fig 12.

EP 3 403 572 A1

R-squared = 0.85

Added variable plot for whole model

* Adjusted data
— Fit: y=0.180502*x
····· 95% conf. bounds

Adjusted y

Adjusted whole model

Performance estimation with four features:

1-slopeDiastolicvar
2-tDiastolicskewness
3-itDiastolicvar
4-firstHalfPointvar

FIG 13

Systolic blod presure estimation with seven features:

1- BRentropy
2-aSystolicskewness
3-itDiastolicskewness
4-tDiffSysDicrskewness
5-tDiffDicrDiasskewness
6-pulseAreatimeDiastolicRatioskewness
7-diastolicDecayskewness

FIG 14.

FIG 15

**Europäisches Patentamt**

**European Patent Office**

**Office européen des brevets**

# EUROPEAN SEARCH REPORT

Application Number

EP 17 17 1597

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 015 067 A1 (MURATA MANUFACTURING CO [JP]; FATIGUE SCIENCE LAB INC [JP]) 4 May 2016 (2016-05-04) * figures 1, 10, 11 * * paragraphs [0061], [0064], [0070], [0096] * | 1-22 | INV. A61B5/024 A61B5/00 A61B5/16 |
| X | EP 2 151 189 A1 (PANASONIC CORP [JP]) 10 February 2010 (2010-02-10) * figure 8 * * paragraphs [0002], [0067] * | 1-22 | |
| X | US 2017/071551 A1 (JAIN JAWAHAR [US] ET AL) 16 March 2017 (2017-03-16) * figure 2A * * paragraphs [0053], [0082], [0149], [0150], [0164], [0167], [0172] * | 1-22 | |
| X | US 2006/247542 A1 (WATANABE YASUYOSHI [JP] ET AL) 2 November 2006 (2006-11-02) * figures 2, 15, 17 * * paragraphs [0083], [0088] - [0091], [0163] * | 1-22 | TECHNICAL FIELDS SEARCHED (IPC) A61B |

The present search report has been drawn up for all claims

| Place of search | Date of completion of the search | Examiner |
|---|---|---|
| The Hague | 6 February 2018 | Almeida, Mariana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 17 1597

This annex lists the patent family members relating to the patent documents cited in the above-mentioned European search report.
The members are as contained in the European Patent Office EDP file on
The European Patent Office is in no way liable for these particulars which are merely given for the purpose of information.

06-02-2018

| Patent document cited in search report | | | Publication date | Patent family member(s) | | | Publication date |
|---|---|---|---|---|---|---|---|
| EP 3015067 | A1 | | 04-05-2016 | CN | 105377137 | A | 02-03-2016 |
| | | | | EP | 3015067 | A1 | 04-05-2016 |
| | | | | JP | 6126220 | B2 | 10-05-2017 |
| | | | | JP | WO2014208289 | A1 | 23-02-2017 |
| | | | | US | 2016106320 | A1 | 21-04-2016 |
| | | | | WO | 2014208289 | A1 | 31-12-2014 |
| EP 2151189 | A1 | | 10-02-2010 | CN | 101677772 | A | 24-03-2010 |
| | | | | CN | 101677773 | A | 24-03-2010 |
| | | | | EP | 2151189 | A1 | 10-02-2010 |
| | | | | EP | 2156788 | A1 | 24-02-2010 |
| | | | | JP | 4964953 | B2 | 04-07-2012 |
| | | | | JP | WO2008149558 | A1 | 19-08-2010 |
| | | | | JP | WO2008149559 | A1 | 19-08-2010 |
| | | | | US | 2010179441 | A1 | 15-07-2010 |
| | | | | US | 2010217137 | A1 | 26-08-2010 |
| | | | | WO | 2008149558 | A1 | 11-12-2008 |
| | | | | WO | 2008149559 | A1 | 11-12-2008 |
| US 2017071551 | A1 | | 16-03-2017 | US | 2017071523 | A1 | 16-03-2017 |
| | | | | US | 2017071537 | A1 | 16-03-2017 |
| | | | | US | 2017071546 | A1 | 16-03-2017 |
| | | | | US | 2017071551 | A1 | 16-03-2017 |
| US 2006247542 | A1 | | 02-11-2006 | JP | 3790266 | B2 | 28-06-2006 |
| | | | | JP | WO2005000119 | A1 | 27-07-2006 |
| | | | | US | 2006247542 | A1 | 02-11-2006 |
| | | | | WO | 2005000119 | A1 | 06-01-2005 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0835633 A2, MARUYA TOKINORI
- US 5381797 A, PAK SONG C
- US 8317716 B, KIM JONG YEOL
- US 20040181159 A, KUO TERRY B J